# EUROPÄISCHE PATENTSCHRIFT

(11) **EP 0 803 510 B1**
(45) Veröffentlichungstag und Bekanntmachung des Hinweises auf die Patenterteilung: **11.09.2002**
(21) Anmeldenummer: 97106064.5
(22) Anmeldetag: 14.04.1997
(51) Int. Cl.: C07F 17/02, C07F 15/00

(54) **2,2'-Disubstituierte 1,1'-Diphosphino-ferrocene und 1',2-disubstituierte 1-Phosphino-ferrocene, Verfahren zu ihrer Herstellung, ihre Verwendung sowie sie enthaltende Übergangsmetallkomplexe**
2,2'-Disubstituted 1,1'-diphosphino ferrocenes and 1',2-disubstituted 1-phosphino ferrocenes, their preparation and use and transition metal complexes containing them
1,1'-Diphosphino-ferrocènes 2,2'-disubstitués et 1-phosphino-ferrocènes 1',2-disubstitués, leur préparation et utilisation et complexes de métaux de transition les contenant

(30) Priorität: 25.04.1996 DE 19616521; 29.01.1997 DE 19703126
(43) Veröffentlichungstag der Anmeldung: 29.10.1997
(73) Patentinhaber: HOECHST AKTIENGESELLSCHAFT, 65929 Frankfurt am Main (DE)
(72) Erfinder: Jendralla, Joachim-Heiner, Dr., 65931 Frankfurt am Main (DE)

(56) Entgegenhaltungen:
- WO-A-96/16971
- TETRAHEDRON LETTERS, Bd. 37, Nr. 1, 1996, Seiten 25-28, XP002031453 SCHWINK, L. ET AL.: "a new practical asymmetric synthesis of c2-symmetrical 1,1'-ferrocenyl diols via cbs-reduction"
- JOURNAL OF THE CHEMICAL SOCIETY, CHEMICAL COMMUNICATIONS, 1989, Seiten 495-496, XP002031454 HAYASHI, T. ET AL.: "a new chiral ferrocenylphosphine ligand with c2 symmetry: preparation and use for palladium-catalysed assymetric cross coupling"

## Beschreibung

Die vorliegende Erfindung betrifft optisch aktive und racemische C₂-symmetrische 2,2'-disubstituierte 1,1'-D.phosphino-ferrocene der Formel Ia, optisch aktive und racemische unsymmetrische 1',2-disubstituierte 1-Phosphino-ferrocene der Formel Ib und achirale 2,2'-disubstituierte 1,1'-Diphosphino-ferrocene der Formel II, Verfahren zu ihrer Herstellung, insbesondere nach dem Prinzip der asymmetrischen ortho-Lithiierung, ihre Verwendung als Liganden für Übergangsmetallkomplexe, sie enthaltende Übergangsmetallkomplexe und die Verwendung der Übergangsmetallkomplexe als Katalysatoren, insbesondere in asymmetrischen Synthesen.

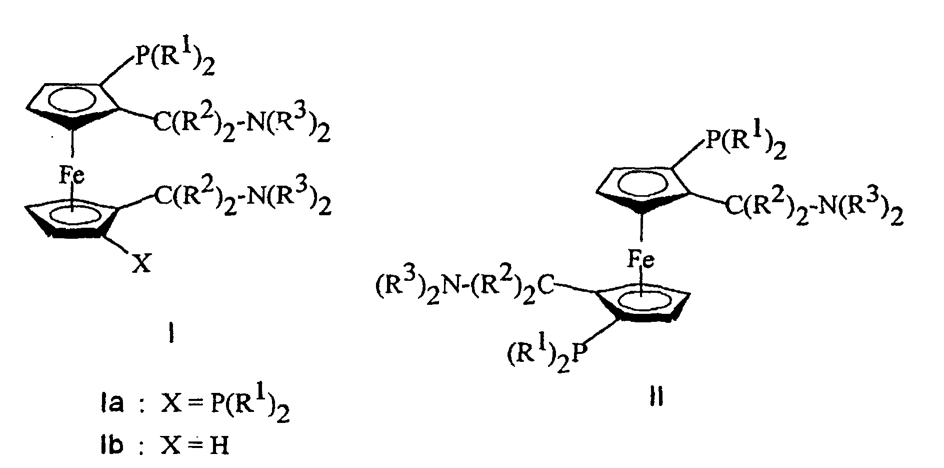

Übergangsmetallkomplexe der homochiralen, unsymmetrischen Mono- bzw. Diphosphino-ferrocene PPFA (Formel A) und BPPFA (Formel B) (in den Formeln bedeuten Me eine Methylgruppe und Ph eine Phenylgruppe) bewirken hohe Stereoselektivität in asymmetrischen Katalysen, wenn das Substrat über eine funktionelle Gruppe verfügt, die mit der Dimethylaminogruppe des Phosphinliganden Wechselwirkungen eingehen kann. Substrate ohne solche funktionelle Gruppen können in asymmetrischen katalytischen Reaktionen mit hoher Enantioselektivität umgesetzt werden, wenn man das homochirale C₂-symmetrische Diphosphino-ferrocen der Formel C als Ligand einsetzt (T. Hayashi et al., J. Am. Chem. Soc. 1994, 116, 4221). Diphosphino-ferrocene des in der Formel C dargestellten Strukturtyps werden beispielsweise beschrieben in T. Hayashi et al., J. Chem. Soc., Chem. Comm., 1989, 495; L. Schwink und P. Knochel, Tetrahedron Lett. 1996, 37, 25; oder WO-A-96/16971. Trotz hervorragender Katalyseergebnisse haben die Liganden der Formeln A, B und C aber keine nennenswerte industrielle Anwendung gefunden, da sie nur mangelhaft zugänglich sind. Zur Herstellung der optisch reinen Liganden ist in jedem Fall eine Racematspaltung notwendig, bei der Synthese der Verbindungen der Formel C zudem eine aufwendige Trennung des chiralen C₂-symmetrischen C von seinem achiralen meso-lsomer. Es besteht somit Bedarf an einfachen Herstellungsverfahren bzw. an geeigneten Liganden, die einfach zugänglich sind.

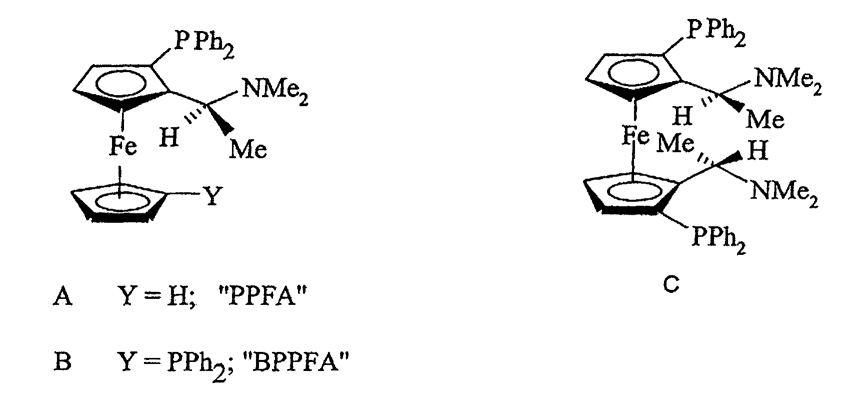

Man hat in jüngster Zeit versucht, homochirale Phosphino-ferrocene durch asymmetrische ortho-Lithiierung substituierter Ferrocene mittels homochiraler Lithiumbasen zu erhalten. So konnte das Dimethylaminomethylferrocen der Formel D mittels 1.5 Äquivalenten n-Butyllithium (n-BuLi) in Gegenwart von 2.0 Äquivalenten des Diamins der Formel E unter Verwendung von 1.5 bis 3.0 Äquivalenten Chlordiphenylphosphin in einer Ausbeute von 49 % und mit bis zu 62 % ee (Enantiomerenüberschuß) zur Verbindung der Formel F diphenylphosphinyliert werden (Y. Nishibayashi et al., J. Org. Chem. 1996, 61, 1172). Auch konnte das N,N-Diisopropylferrocencarboxamid der Formel G mittels 2.2 Äquivalenten n-BuLi und 2.2 Äquivalenten des Diamins der Formel H unter Verwendung von 3.0 Äquivalenten Chlordiphenylphosphin in einer Ausbeute von 82 % und mit 90 % ee zur Verbindung der Formel J diphenylphosphinyliert werden (M. Tsukazaki, V. Snieckus et al., J. Am. Chem. Soc. 1996, 118, 685).

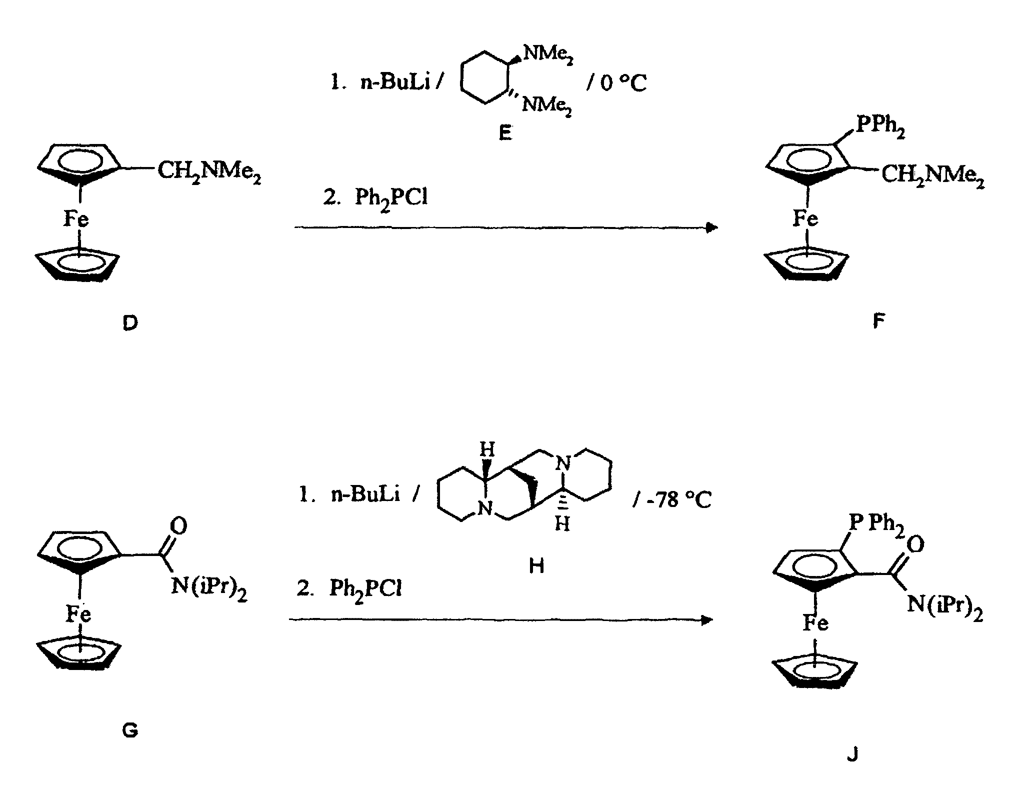

Während somit eine asymmetrische ortho-Lithiierung an einem Ring des Ferrocens bekannt ist, gibt es keine Hinweise, daß dieses Prinzip auf beide Ringe ausgedehnt werden könnte; es wurde noch nie versucht, die Methode der asymmetrischen ortho-Lithiierung mittels einer optisch aktiven Lithiumbase auf beide Ferrocenringe anzuwenden.

Überraschend wurde nun ein sehr einfaches Verfahren zur Herstellung von Phosphino-ferrocenen gefunden, das auf dem Prinzip der asymmetrischen ortho-Lithiierung beruht, die hier erstmalig auf beide Ferrocenringe angewendet wird und erstmalig zur asymmetrischen Synthese chiraler C₂-symmetrischer Diphosphino-ferrocene dient. Ausgehend beispielsweise von der kommerziell verfügbaren 1,1'-Ferrocendicarbonsäure sind danach auf einem extrem kurzen Syntheseweg neue Monophosphino- und Diphosphino-ferrocene sehr einfach zugänglich, die sich als Liganden für katalytisch aktive Übergangsmetallkomplexe eignen. Durch Variation des erfindungsgemäßen Verfahrens sind so wahlweise unter anderem optisch aktive 1',2-disubstituierte 1-Phosphino-ferrocene der Formel Ib oder optisch aktive 2,2'-disubstituierte 1,1'-Diphosphino-ferrocene der Formel la oder, in hochdiastereoselektiver Weise, die achiralen meso-lsomeren der Diphosphine la, d. h. die Verbindungen der Formel II, bequem erhältlich.

Gegenstand der vorliegenden Erfindung sind somit zum einen Verbindungen der Formel I, 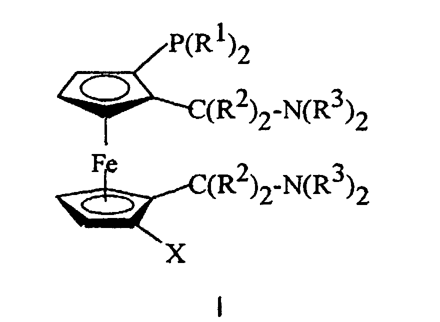 worin
die Substituenten R¹ Cyclohexyl, unsubstituiertes Phenyl C₆H₅ oder substituiertes Phenyl C₆H₅₋ₙR⁴ₙ bedeuten, wobei n für 1 bis 5 steht und die Substituenten R⁴ unverzweigtes oder verzweigtes C₁- bis C₄-Alkyl, C₁- bis C₄-Alkoxy oder Halogen bedeuten;
die beiden geminalen Substituenten R² zusammen ein doppelt gebundenes Sauerstoffatom bedeuten (d. h. (R²)₂ bedeutet =O) oder jeder Substituent R² für sich Wasserstoff bedeutet;
die Substituenten R³ jeder für sich unverzweigtes oder verzweigtes C₁- bis C₄-Alkyl, Cyclopentyl, Cyclohexyl, unsubstituiertes Phenyl C₆H₅ oder substituiertes Phenyl C₆H₅₋ₙR⁴ₙ bedeuten, wobei n und R⁴ die oben unter R¹ angegebenen Bedeutungen haben, oder die Substituenten R³ miteinander unter Ausbildung eines Ringes verbunden sind, wobei sie zusammen Tetramethylen -(CH₂)₄-, Pentamethylen -(CH₂)₅-, 3-Oxa-pentamethylen -(CH₂)₂-O-(CH₂)₂- oder N-Methyl-3-azapentamethylen -(CH₂)₂-N(CH₃)-(CH₂)₂- bedeuten;
der Substituent X Wasserstoff oder P(R¹)₂ bedeutet; und ihre Salze.

Die in der Formel I sowie in anderen Formeln, z. B. der Formel II, dargestellten Konformationen entsprechen nicht unbedingt den Konformationen, in der die erfindungsgemäßen Verbindungen unter bestimmten Bedingungen tatsächlich vorliegen und die sich z. B. aus den Röntgenstrukturanalysen ergeben. Die Formeln dienen nur zur Darstellung der Substituentenanordnung am Ferrocensystem und sind nicht als einschränkend hinsichtlich einer bestimmten sterischen Anordnung zu verstehen. Zu verschiedenen Konformationen der erfindungsgemäßen Verbindungen gelangt man u. a. insbesondere durch Verdrehen eines der beiden Ferrocenringe um eine durch die Mittelpunkte beider Ringe verlaufende Drehachse. Welche Konformation hinsichtlich dieser Achse tatsächlich vorliegt, hängt vom Einzelfall ab. Die Erfindung umfaßt natürlich alle Verbindungen der Formeln I und II (und deren Komplexe) unabhängig von der jeweils zeichnerisch dargestellten Konformation oder Konfiguration.

Die vorliegende Erfindung umfaßt zum einen die freien Verbindungen der Formel I, d. h. die Verbindungen, die keine Salze sind. Die Erfindung umfaßt aber zum anderen auch die Salze von Verbindungen der Formel I. Z. B. können Verbindungen der Formel I, insbesondere solche, die basische Gruppen enthalten, also beispielsweise Verbindungen, in denen die Reste R² Wasserstoff bedeuten, mit anorganischen und organischen Säuren, insbesondere mit nicht-oxidierenden Säuren, Säureadditionssalze bilden. Als Säuren kommen z. B. Chlorwasserstoffsäure, Bromwasserstoffsäure, lodwasserstoffsäure, Schwefelsäure, Salpetersäure, Phosphorsäure, Essigsäure, Trifluoressigsäure, Methansulfonsäure, u. a. m. in Betracht. Die Salze können auf die übliche Weise, z. B. durch Vereinigen der Komponenten in einem Lösungs- oder Verdünnungsmittel, hergestellt werden. Generell umfaßt die Erfindung die Verbindungen der Formel II auch dann, wenn sie in Form von Addukten mit anderen Molekülen oder Ionen oder Atomen vorliegen, wie dies beispielsweise in Komplexen mit Metallionen oder Säure-Base-Addukten der Fall ist.

Die Erfindung umfaßt alle stereoisomeren Formen der Verbindungen der Formel I, also alle enantiomeren und diastereomeren Formen, und insbesondere umfaßt sie sowohl das eine als auch das andere Enantiomer. Die Verbindungen der Formel I können neben der durch die Substituentenanordnung am Ferrocen bedingten Chiralität keine weiteren Chiralitätselemente aufweisen, es können jedoch z. B. auch zusätzlich in den Resten R¹ und R³ asymmetrische Kohlenstoffatome vorliegen. Diese können dann unabhängig voneinander die R- oder die S-Konfiguration aufweisen. Enantiomere werden sowohl in Form reiner Enantiomerer als auch in Form des Racemats oder in Form von Gemischen der Enantiomeren in beliebigen Mengenverhältnissen umfaßt, insbesondere in Form von Gemischen, die einen hohen Enantiomerenüberschuß aufweisen. Ebenso werden auch alle Diastereomeren in reiner Form und in Gemischen mit beliebigen Mengenverhältnissen umfaßt.

Die Erfindung umfaßt ebenso Gemische von zwei oder mehr Verbindungen der Formel I, die sich in der Summenformel unterscheiden, insbesondere Gemische aus Verbindungen der Formel I, in der X für Wasserstoff steht, und den entsprechenden Verbindungen der Formel I, in der X für P(R¹)₂ steht, wobei wiederum alle Verbindungen in allen stereoisomeren Formen vorliegen können.

Beispiele für C₁- bis C₄-Alkyl sind Methyl, Ethyl, n-Propyl, Isopropyl, n-Butyl, sec-Butyl, Isobutyl und tert-Butyl, Beispiele für C₁- bis C₄-Alkoxy sind Methoxy, Ethoxy, n-Propoxy, Isopropoxy, n-Butoxy, sec-Butoxy, Isobutoxy und tert-Butoxy. In substituierten Phenylresten können sich die Substituenten in beliebigen Positionen befinden, bei Monosubstitution in der ortho-, meta- oder para-Position, bei Disubstitution beispielsweise in der 3,4- oder 3,5-Position. Sind Phenylringe mehrfach durch Reste R⁴ substituiert, so können die Reste R⁴ gleich oder verschieden sein. Die Zahl n der Substituenten in Phenylringen kann 1, 2, 3, 4 oder 5 sein. Unter den substituierten Phenylresten sind monosubstituierte Phenylringe bevorzugt.

Halogen bedeutet, soweit nicht anders angegeben, Fluor, Chlor, Brom oder lod.

Die beiden Reste R¹ in der Gruppe P(R¹)₂ in den Verbindungen der Formel I können gleich oder verschieden sein. In einer bevorzugten Ausführungsform der Erfindung sind sie gleich. Auch die beiden Reste R³ am Stickstoffatom können, wenn sie nicht miteinander unter Ausbildung eines Ringes verbunden sind, gleich oder verschieden sein. Sind die beiden Reste R³ untereinander unter Ausbildung eines Ringes verbunden, so ist darunter zu verstehen, daß die beiden an dasselbe Stickstoffatom gebundenen Reste R³ untereinander verbunden sind und der Rest N(R³)₂ somit für Pyrrolidino, Piperidino, Morpholino oder N-Methylpiperazino steht.

Bevorzugt steht R¹ in den Verbindungen der Formel I für Cyclohexyl, Phenyl, p-Tolyl, p-tert-Butylphenyl oder p-Halogenophenyl, wobei Halogen hier für Fluor, Chlor oder Brom steht. Besonders bevorzugt steht R¹ für Phenyl.

Bevorzugt stehen die beiden Reste R² in den Verbindungen der Formel I zusammen für ein doppelt gebundenes Sauerstoffatom (d. h. (R²)₂ bedeutet =O).

Bevorzugt steht R³ in den Verbindungen der Formel I für Isopropyl, Cyclohexyl oder Phenyl oder die beiden Reste R³ stehen zusammen für -(CH₂)₄-, -(CH₂)₅-, -(CH₂)₂-O-(CH₂)₂- oder -(CH₂)₂-N(CH₃) -(CH₂)₂-, besonders bevorzugt steht R³ für Isopropyl.

Bevorzugt steht X in den Verbindungen der Formel I für Wasserstoff oder für eine Gruppe P(R¹)₂, in der R¹ die angegebenen bevorzugten Bedeutungen hat.

Bevorzugte Verbindungen der Formel I sind solche, in denen einer oder mehrere der Substituenten bevorzugte Bedeutungen haben. Besonders bevorzugt sind Verbindungen der Formel I, in der
die Substituenten R¹ Cyclohexyl, Phenyl, p-Tolyl, p-tert-Butylphenyl oder p-Halogenophenyl bedeuten, wobei Halogen hier für Fluor, Chlor oder Brom steht;
die beiden Substituenten R² zusammen ein doppelt gebundenes Sauerstoffatom bedeuten (d. h. (R²)₂ bedeutet =O) oder jeder Substituent R² für sich Wasserstoff bedeutet;
die Substituenten R³ Isopropyl, Cyclohexyl oder Phenyl bedeuten oder die beiden Substituenten R³ zusammen für -(CH₂)₄-, -(CH₂)₅-, -(CH₂)₂-O-(CH₂)₂- oder -(CH₂)₂-N(CH₃)-(CH₂)₂- stehen;
der Substituent X Wasserstoff oder P(R¹)₂ bedeutet, wobei R¹ für Cyclohexyl, Phenyl, p-Tolyl, p-tert-Butylphenyl oder p-Halogenophenyl steht und Halogen hier für Fluor, Chlor oder Brom steht.

Ganz besonders bevorzugt sind Verbindungen der Formel I, in der R¹ für Phenyl steht, die beiden Substituenten R² zusammen für ein doppelt gebundenes Sauerstoffatom stehen (d. h. (R²)₂ bedeutet =O), R³ für Isopropyl steht und X für Wasserstoff oder P(Phenyl)₂ steht.

Darüber hinaus bevorzugt sind einerseits die Verbindungen der Formel I in optisch aktiver Form und andererseits die Verbindungen der Formel I in racemischer Form.

Für die bevorzugten Verbindungen gelten die obigen Erläuterungen entsprechend, auch hier werden - soweit zutreffend - alle stereoisomeren Formen und deren Gemische in beliebigen Verhältnissen sowie die Salze der Verbindungen umfaßt.

Gegenstand der vorliegenden Erfindung sind weiterhin Verbindungen der Formel II, 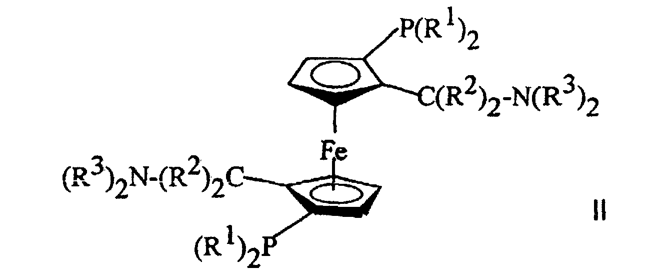 worin
die Substituenten R¹ Cyclohexyl, unsubstituiertes Phenyl C₆H₅ oder substituiertes Phenyl C₆H₅₋ₙR⁴ₙ bedeuten, wobei n für 1 bis 5 steht und die Substituenten R⁴ unverzweigtes oder verzweigtes C₁- bis C₄-Alkyl, C₁- bis C₄-Alkoxy oder Halogen bedeuten;
die beiden geminalen Substituenten R² zusammen ein doppelt gebundenes Sauerstoffatom bedeuten (d. h. (R²)₂ bedeutet =O) oder jeder Substituent R² für sich Wasserstoff bedeutet;
die Substituenten R³ jeder für sich unverzweigtes oder verzweigtes C₁- bis C₄-Alkyl, Cyclopentyl, Cyclohexyl, unsubstituiertes Phenyl C₆H₅ oder substituiertes Phenyl C₆H₅₋ₙR⁴ₙ bedeuten, wobei n und R⁴ die oben unter R¹ angegebenen Bedeutungen haben, oder die Substituenten R³ miteinander unter Ausbildung eines Ringes verbunden sind, wobei sie zusammen Tetramethylen -(CH₂)₄-, Pentamethylen -(CH₂)₅-, 3-Oxa-pentamethylen -(CH₂)₂-O-(CH₂)₂- oder N-Methyl-3-azapentamethylen -(CH₂)₂-N(CH₃)-(CH₂)₂- bedeuten; und ihre Salze.

Wie bei den Verbindungen der Formel I umfaßt die vorliegende Erfindung zum einen die freien Verbindungen der Formel II, d. h. die Verbindungen, die keine Salze sind. Die Erfindung umfaßt aber zum anderen auch die Salze von Verbindungen der Formel II. Z. B. können Verbindungen der Formel II, insbesondere solche, die basische Gruppen enthalten, also beispielsweise Verbindungen, in denen die Reste R² Wasserstoff bedeuten, mit anorganischen und organischen Säuren, insbesondere mit nicht-oxidierenden Säuren, Säureadditionssalze bilden. Als Säuren kommen z. B. Chlorwasserstoffsäure, Bromwasserstoffsäure, Iodwasserstoffsäure, Schwefelsäure, Salpetersäure, Phosphorsäure, Essigsäure, Trifluoressigsäure, Methansulfonsäure, u. a. m. in Betracht. Die Salze können auf die übliche Weise, z. B. durch Vereinigen der Komponenten in einem Lösungs- oder Verdünnungsmittel, hergestellt werden. Generell umfaßt die Erfindung die Verbindungen der Formel II auch dann, wenn sie in Form von Addukten mit anderen Molekülen oder Ionen oder Atomen vorliegen, wie dies beispielsweise in Komplexen mit Metallionen oder Säure-Base-Addukten der Fall ist.

Die achiralen Verbindungen der Formel II stellen die meso-Isomeren zu den chiralen Verbindungen der Formel la dar. Gegenstand der vorliegenden Erfindung sind auch Gemische, die Verbindungen der Formel II und Verbindungen der Formel I in beliebigen Mengenverhältnissen enthalten. Die oben zu den Verbindungen der Formel I gegebenen Erläuterungen zu stereoisomeren Formen und Gemischen gelten hier entsprechend, die Verbindungen können also wiederum in allen stereoisomeren Formen vorliegen, und die Mischungen können zwei oder mehr Verbindungen enthalten, so z. B. nebeneinander Verbindungen der Formeln la, Ib und II.

Die obigen Erläuterungen zu den Resten in den Verbindungen der Formel I, z. B. Alkylresten, Alkoxyresten, Phenylresten oder Halogen oder der Zahl n, gelten ebenso für die Reste in den Verbindungen der Formel II. Ebenso gelten die obigen Ausführungen zu den bevorzugten Bedeutungen der Reste R¹, R² und R³ und zu den bevorzugten Verbindungen der Formel I entsprechend für die Verbindungen der Formel II.

Gegenstand der vorliegenden Erfindung sind auch Verfahren zur Herstellung von Verbindungen der Formeln I und II. Insbesondere sind Gegenstand der Erfindung Verfahren zur Herstellung von Verbindungen der Formel la und Ib in optisch aktiver Form, insbesondere in hoher optischer Reinheit. Diese Verfahren beinhalten bei der Herstellung von Verbindungen der Formel la eine zweifache asymmetrische ortho-Lithiierung bzw. bei der Herstellung von Verbindungen der Formel Ib eine einfache asymmetrische ortho-Lithiierung. Mittels der erfindungsgemäßen Verfahren können durch Variation von Natur und Menge der Lithiumbase, die chiral oder achiral sein kann, und durch Modifizierung der genauen Lithiierungs-Prozedur mit sehr hoher Chemoselektivität, Diastereoselektivität und Enantioselektivität gezielt z. B. die optisch aktiven C₂-symmetrische Diphosphine der Formel la oder die achiralen meso-lsomeren der Diphosphine der Formel Ia, d. h. die Verbindungen der Formel II, oder die optisch aktiven unsymmetrischen Monophosphine der Formel Ib hergestellt werden. Die Erfindung umfaßt aber auch die analoge Herstellung der entsprechenden racemischen Verbindungen der Formel I.

Als direktes Ausgangsmaterial für das erfindungsgemäße Herstellungsverfahren können Verbindungen der Formel III, 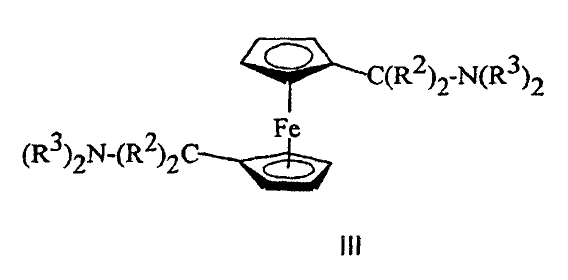 dienen, in der die Substituenten R² und R³ die oben angegebenen Bedeutungen haben. Die Verbindungen der Formel III lassen sich aus kommerziell erhältlicher 1,1'-Ferrocendicarbonsäure erhalten. Diese kann einstufig mit hohen Ausbeuten in literaturbekannter Weise zu Ferrocendiamiden der Formel III umgesetzt werden, in der die beiden geminalen Reste R² zusammen für ein doppelt gebundenes Sauerstoffatom stehen. Die Amidreste R³ können hierbei in literaturbekannter Weise innerhalb des gesamten oben angegebenen Bedeutungsbereichs von R³ variiert werden, wozu ausschließlich die Variation des durchweg kommerziell erhältlichen Amins HN(R³)₂ notwendig ist (P.J. Hammond et al., J. Organomet. Chem. 1986, 306, 367 ; P.D. Beer et al., J. Organomet. Chem. 1988, 350, C15 ; J.C. Medina et al., J. Am. Chem. Soc. 1991, 113, 365; J.T. Yli-Kauhaluoma et al., J. Am. Chem. Soc. 1995, 117, 7041; W. Zhang et al., Tetrahedron: Asymmetry 1996, 7, 451). Die Diamide der Formel III, d. h. die Verbindungen der Formel III, in der die beiden Reste R² zusammen für ein doppelt gebundenes Sauerstoffatom stehen, können gemäß literaturbekannten Verfahren mit verschiedenen Reduktionsmitteln, bevorzugt mit dem Boran-Tetrahydrofuran-Komplex, in hohen Ausbeuten zu den entsprechenden Diaminen der Formel III reduziert werden, in der jeder Rest R² für sich Wasserstoff bedeutet.

Es wurde nun gefunden, daß die Umsetzung von Verbindungen der Formel III, in denen die Substituenten R² und R³ die oben angegebenen Bedeutungen haben, mit n-Butyllithium in Gegenwart tertiärer Amine, bevorzugt chelatisierender tertiärer Diamine, selbst bei Anwendung großer Überschüsse der Lithiumbase (> 4 Äquivalente relativ zum Substrat III) unter fast vollständiger Mono-ortho-Lithiierung verläuft. Setzt man dem Reaktionsgemisch dann ein Chlorphosphin der Formel CIP(R¹)₂ zu, in der die Reste R¹ die oben angegebenen Bedeutungen haben und die nach oder analog zu bekannten Verfahren erhältlich sind und die auch überwiegend kommerziell erhältlich sind, so erhält man in hohen Ausbeuten die Verbindungen der Formel Ib. Wird die Lithiumbase durch Umsetzung von n-Butyllithium mit einem optisch reinen (homochiralen) tertiären Amin, bevorzugt einem optisch reinen chelatisierenden tertiären Diamin, erhalten, so entstehen die Verbindungen der Formel Ib mit hoher Enantioselektivität. Wie sich aus den Beispielen ergibt, ist diese unter geeigneten Reaktionsbedingungen so hoch (>80 % ee (ee = Enantiomerenüberschuß)), daß die Verbindungen der Formel Ib direkt oder z. B. nach nur einmaliger Umkristallisation in guten Ausbeuten in optisch reiner Form (≥98 % ee) erhalten werden können. Wird die Lithiumbase durch Umsetzung von n-Butyllithium mit einem racemischen oder einem achiralen tertiären Amin erhalten, so entstehen unter ansonsten gleichen Reaktionsbedingungen die Verbindungen der Formel Ib in racemischer Form.

Gegenstand der vorliegenden Erfindung ist somit ein Verfahren zur Herstellung von optisch aktiven Monophosphinen der Formel I, in der die Reste R¹, R² und R³ die oben angegebenen Bedeutungen haben und X für Wasserstoff steht, dadurch gekennzeichnet, daß man Verbindungen der Formel III, 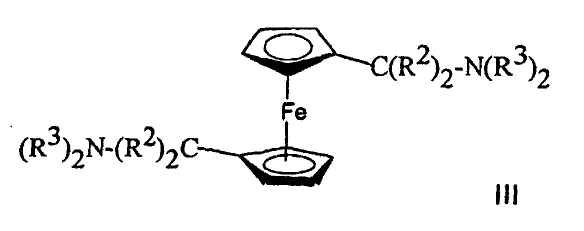 in der die Reste R² und R³ die oben angegebenen Bedeutungen haben, mit n-Butyllithium in Gegenwart eines homochiralen tertiären Amins asymmetrisch mono-ortho-lithiiert und die chirale Monolithiumverbindung in situ mit einem Chlorphosphin der Formel CIP(R¹)₂ umsetzt, in der die Reste R¹ die oben angegebene Bedeutung haben.

Gegebenenfalls wird anschließend die optische Reinheit des Produkts der Formel I noch durch Umkristallisation gesteigert. Die optische Reinheit des nach dem erfindungsgemäßen Verfahren zunächst erhaltenen Produkts der Formel I hängt vom Einzelfall ab, z. B. von den Substituenten und den Reaktionsbedingungen. Vielfach ist sie bereits so hoch, daß für die vorgesehene Verwendung keine Steigerung erforderlich ist. Wenn eine Steigerung der optischen Reinheit gewünscht wird, kann dies aber z. B. durch Umkristallisation unter dem Fachmann geläufigen Bedingungen erfolgen, wobei vielfach bereits eine Umkristallisation ausreichend ist.

Die asymmetrische Reaktion kann im einzelnen z. B. in folgender Weise durchgeführt werden. Das optisch reine tertiäre Amin, bevorzugt ein optisch reines chelatisierungsfähiges tertiäres Diamin, wird in einem inerten, nicht oder nur schwach koordinierenden Lösungsmittel in einer Inertgasatmosphäre (bevorzugt Stickstoff oder Argon) vorgelegt, bevorzugt bei einer Temperatur von -78 °C bis Raumtemperatur, besonders bevorzugt -78 °C bis 0 °C, ganz besonders bevorzugt bei einer Temperatur von -78 °C bis -40 °C. Besonders bevorzugt als Amine sind Diamine wie z. B. Spartein, trans-N,N,N',N'-Tetramethyl-1,2-cyclohexan-diamin, N,N,N',N'-Tetramethyl-1,2-diphenylethylendiamin, 1-Methyl-2-(piperidinomethyl)-pyrrolidin, O-Alkyl-dihydrochinidine oder O-Alkyl-dihydrocinchonidine. Ganz besonders bevorzugt ist Spartein. Die Amine können als (+)-Isomer oder als (-)-Isomer eingesetzt werden. Das Amin wird bevorzugt in einer Menge von 1.2 bis 5 Äquivalenten, bezogen auf das Edukt der Formel III, eingesetzt, besonders bevorzugt in einer Menge von 1.5 bis 4 Äquivalenten, ganz besonders bevorzugt 1.5 bis 2 Äquivalenten. Bevorzugte Lösungsmittel sind aliphatische und aromatische Kohlenwasserstoffe und Ether, besonders bevorzugt sind Toluol, Diethylether, Methyl-tert-Butylether und Diisopropylether. Zum Amin wird dann eine Lösung von n-Butyllithium in einem inerten Lösungsmittel, bevorzugt eine 1- bis 10-molare Lösung in Pentan, Hexan oder Cyclohexan, besonders bevorzugt eine 1.6- bis 2.5-molare Lösung in Hexan zugegeben. Die Menge des n-Butyllithiums beträgt bevorzugt 1.2 bis 5 Äquivalente, bezogen auf das Edukt der Formel III, besonders bevorzugt 1.5 bis 4 Äquivalente, ganz besonders bevorzugt 1.5 bis 2 Äquivalente. Es ist bevorzugt, daß die molare Menge n-Butyllithium gleich oder etwas geringer als die des chiralen Amins ist, so daß sämtliches n-Butyllithium assoziert mit dein chiralen Amin vorliegt. Nach kurzem Nachrühren wird das Edukt der Formel III, entweder als Reinsubstanz oder z. B. gelöst in einem der oben genannten Lösungsmittel, zugegeben. Die Zugabegeschwindigkeit ist unkritisch. Die Reaktionstemperatur wird durch Kühlung aufrechterhalten. Nach einem Zeitraum von üblicherweise 15 Minuten bis 2 Stunden (der optimale Zeitraum ist umso kürzer, je größer der Überschuß an n-Butyllithium und chiralem Amin ist) gibt man das Chlorphosphin der Formel CIP(R¹)₂ als Reinsubstanz oder z. B. als Lösung in einem der oben genannten Lösungsmittel zu. Die Zugabegeschwindigkeit ist weitgehend unkritisch. Bevorzugt ist es, flüssige Chlorphosphine als Reinsubstanz innerhalb 5 bis 15 Min. zuzutropfen, damit die Reaktionstemperatur leicht durch Kühlung aufrechterhalten werden kann. Man läßt im allgemeinen 15 bis 90 Min. nachrühren. Die optimale Reaktionszeit hängt vom Lösungsmittel und von den Substituenten R¹, R² und R³ ab und kann leicht durch Entnahme einer Probe und beispielsweise dünnschichtchromatographische Untersuchung anhand des Verschwindens der Verbindung der Formel III und der Bildung der Verbindung der Formel Ib ermittelt werden. Zur Isolierung des Produkts erfolgt bevorzugt eine wäßrige Aufarbeitung des Reaktionsgemisches unter sauren Bedingungen. So kann nach vollständiger Reaktion zur Aufarbeitung das Reaktionsgemisch beispielsweise zunächst auf 0 bis 25 °C oder auf Raumtemperatur erwärmt werden und dann beispielsweise mit einem Überschuß einer gesättigten wäßrigen Lösung von Ammoniumchlorid (NH₄Cl) versetzt werden. Man kann aber beispielsweise eine Ammoniumchloridlösung auch schon bei tieferer Temperatur zusetzen und erst dann erwärmen. Die Resultate dieser beiden Durchführungsweisen unterscheiden sich nur geringfügig. Die wäßrige Standardaufarbeitung z. B. mit Extraktion liefert schließlich das optisch aktive Monophosphin der Formel Ib. Die erhaltenen Produkte sind als isolierter Feststoff kaum, in Lösung aber mäßig luftempfindlich (Bildung der Phosphinoxide). Die Extraktionen werden deshalb bevorzugt in einer Inertgasatmosphäre bzw. unter Minimierung des Zeitraums der Lufteinwirkung vorgenommen.

Wie man den Beispielen entnimmt, erhält man so beispielsweise für das Edukt der Formel III, in der die Reste R² zusammen für ein doppelt gebundenes Sauerstoffatom stehen und R³ für Isopropyl steht, selbst mit 4.40 Äquivalenten n-Butyllithium und 4.45 Äquivalenten (-)-Spartein in Ether bei -70 bis -78 °C bei einer Lithiierungszeit von 1 Stunde und unter Verwendung von Chlordiphenylphosphin mehr als 85 % des Monophosphins der Formel Ib und weniger als 15 % der Diphosphine der Formeln II und/oder la. Das Rohprodukt hatte dabei 80 % ee und kann nach Standardreinigungsverfahren weiter gereinigt werden. Im betrachteten Beispiel wird beispielsweise nach Umkristallisation aus heißem n-Heptan die analytisch reine Verbindung der Formel Ib in 58 % Ausbeute und mit 98.5 % ee erhalten. Die optische Reinheit läßt sich durch HPLC auf chiraler Phase zuverlässig ermitteln. Bei einem verringerten Überschuß an n-Butyllithium/Spartein und einer verkürzten Lithiierungszeit steigt der Anteil des Monophosphins der Formel Ib im Rohprodukt weiter an. Wie man weiterhin den Beispielen entnimmt, erhält man für das gleiche Edukt der Formel III (mit den gleichen Bedeutungen der Reste R² und R³) mit 1.9 Äquivalenten n-Butyllithium und 2.0 Äquivalenten (-)-Spartein in Toluol bei -70 bis -78 °C bei einer Lithiierungszeit von 45 Min. und unter Verwendung von Chlordiphenylphosphin mehr als 93 % des Monophosphins der Formel Ib, das bereits als Rohprodukt 91 % ee hat. Einmalige Umkristallisation liefert dann das optisch reine (99 % ee) Produkt der Formel Ib in 75 % Ausbeute.

Wird die obige Reaktion in Ether als Lösungsmittel mit dem achiralen Diamin N,N,N',N'-Tetramethylethylendiamin (TMEDA) anstatt mit einem optisch aktiven Amin wie Spartein unter ansonsten gleichen Bedingungen wie vorstehend beispielhaft beschrieben durchgeführt, so wird die racemische Verbindung der Formel Ib in 79 % Ausbeute erhalten.

Führt man die asymmetrische Lithiierung/Phosphinylierung von Substraten der Formel III unter gleichen Bedingungen wie oben beschrieben, aber unter Einsatz von sec-Butyllithium anstatt n-Butyllithium durch, so erhält man mit hoher Chemoselektivität und Diastereoselektivität die entsprechenden meso-Diphosphine der Formel II. Chirale C₂-symmetrische Diphosphine der Formel la und Monophosphine der Formel Ib werden unter diesen Bedingungen selbst dann nur in sehr geringen Mengen gebildet, wenn man den Überschuß der Lithiumbase auf 3.0 Äquivalente verringert. Die hohe Diastereoselektivität zugunsten der Verbindungen der Formel II gegenüber denen der Formel la ist völlig überraschend. Obwohl die beiden ortho-Lithiierungen am oberen und am unteren Ring des Ferrocens ja von der gleichen homochiralen Lithiumbase bewirkt werden müssen, verläuft mit sec-Butyllithium die ortho-Lithiierung/Phosphinylierung in Summe am unteren Ferrocenring mit entgegengesetzter Enantioselektivität zu der am oberen Ferrocenring.

Gegenstand der vorliegenden Erfindung ist somit weiterhin ein Verfahren zur Herstellung von Verbindungen der Formel II, in der die Reste R¹, R² und R³ die oben angegebenen Bedeutungen haben, dadurch gekennzeichnet, daß man Verbindungen der Formel III, 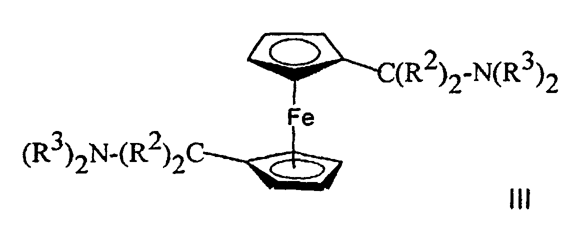 in der die Reste R² und R³ die oben angegebenen Bedeutungen haben, mit sec-Butyllithium in Gegenwart eines tertiären Amins, bevorzugt in Gegenwart eines chelatisierungsfähigen tertiären Diamins, besonders bevorzugt in Gegenwart eines achiralen oder racemischen chelatisierungsfähigen tertiären Diamins, beispielsweise in Gegenwart von N,N,N',N'-Tetramethylethylendiamin (TMEDA), ortho-lithiiert und die Dilithiumverbindung in situ mit einem Chlorphosphin der Formel CIP(R¹)₂ umsetzt, in der die Reste R¹ die oben angegebene Bedeutung haben.

Die oben zum Verfahren zur Herstellung von Verbindungen der Formel Ib gegebenen Erläuterungen, betreffend z. B. die Amine, die Lösungsmittel, die Mengen oder die Reaktionstemperaturen, gelten hier entsprechend. Dabei ist, wenn wie hier in einem Herstellungsverfahren eine zweifache Lithiierung/Phosphinylierung des Ferrocens angestrebt wird, naturgemäß die Menge an Lithiierungsreagenz, die mindestens eingesetzt wird, größer und beträgt mindestens 2 Äquivalente, bezogen auf das Edukt der Formel III. Die meisten Diphosphine der Formel II sind in Wasser praktisch unlöslich und in den oben genannten Reaktionslösungsmitteln nur schlecht bis sehr mäßig löslich. Wird das Reaktionsgemisch, wie oben beispielhaft beschrieben, durch Zugabe einer wäßrigen Ammoniumchloridlösung aufgearbeitet, so fallen die Verbindungen der Formel II aus und können häufig durch einfaches Absaugen isoliert werden.

Wie man den Beispielen entnimmt, erhält man so beispielsweise für das Edukt der Formel III, in der die Reste R² zusammen für ein doppelt gebundenes Sauerstoffatom stehen und R³ für Isopropyl steht, mit sec-Butyllithium und (-)-Spartein in Ether bei -70 bis -78 °C bei einer Lithiierungszeit von 2 Stunden und unter Verwendung von Chlordiphenylphosphin ein Rohprodukt mit einem Verhältnis Verbindung II: Verbindung la von ca. 95 : 5. Dieses Verhältnis wurde übereinstimmend nach verschiedenen Methoden ermittelt. Das reine meso-Diphosphin wird nach Umkristallisation in 75 % Ausbeute erhalten. Außer durch Spektren ist die Struktur der Verbindung der Formel II durch eine Einkristall-Röntgenstrukturanalyse gesichert. Wird das achirale Diamin TMEDA anstatt Spartein eingesetzt, so erhält man das meso-Diphosphin der Formel II bei unverändert hoher Diastereoselektivität in 64 % Ausbeute.

Führt man die asymmetrische Dilithiierung/Diphosphinylierung der Edukte der Formel III hingegen nicht auf einmal, sondern schrittweise durch, indem man die wie oben beschrieben erhaltenen optisch aktiven Monophosphine der Formel Ib erneut den gleichen Bedingungen unterwirft, unter denen sie gebildet wurden, so erhält man bei praktisch quantitativem Umsatz der Monophospine der Formel Ib die optisch reinen chiralen, C₂-symmetrischen Diphosphine der Formel la mit sehr hoher Diastereoselektivität. Die beiden schrittweisen Monolithiierungen/Monophosphinylierungen, induziert durch eine aus n-Butyllithium und einem homochiralen tertiären Amin gebildete chirale Lithiumbase, erfolgen somit mit der gleichen Enantioselektivität am unteren und am oberen Ferrocenring.

Gegenstand der vorliegenden Erfindung ist damit auch ein Verfahren zur Herstellung von optisch aktiven C₂-symmetrischen Diphosphinen der Formel I, in der die Reste R¹, R² und R³ die oben angegebenen Bedeutungen haben und X für P(R¹)₂ steht, dadurch gekennzeichnet, daß man die optisch aktiven Monophosphine der Formel I, in der die Reste R¹, R² und R³ die oben angegebenen Bedeutungen haben und X für Wasserstoff steht, mit n-Butyllithium in Gegenwart eines homochiralen tertiären Amins asymmetrisch mono-ortho-lithiiert und die chirale Monolithiumverbindung in situ mit einem Chlorphosphin der Formel CIP(R¹)₂ umsetzt, in der die Reste R¹ die oben angegebene Bedeutung haben.

Gegebenenfalls wird auch hier anschließend die optische Reinheit des Produkts der Formel I noch durch Umkristallisation gesteigert. Die optische Reinheit des nach dem erfindungsgemäßen Verfahren zunächst erhaltenen Produkts der Formel I hängt wiederum vom Einzelfall ab, z. B. von den Substituenten und den Reaktionsbedingungen. Auch hier ist vielfach die optische Reinheit des nach dem erfindungsgemäßen Verfahren direkt erhaltenen Produkts bereits so hoch, daß für die vorgesehene Verwendung keine weitere Steigerung erforderlich ist. Wenn eine Steigerung der optischen Reinheit gewünscht wird, kann diese durch Umkristallisation unter dem Fachmann geläufigen Bedingungen erfolgen, wobei vielfach bereits eine Umkristallisation ausreichend ist.

Die oben zum Verfahren zur Herstellung von Verbindungen der Formel Ib gegebenen Erläuterungen, betreffend z. B. die Amine, die Lösungsmittel, die Mengen oder die Reaktionstemperaturen, gelten hier entsprechend. Ähnlich den meso-Diphosphinen der Formel II sind auch die meisten C₂-symmetrischen Diphosphine der Formel la in Wasser praktisch unlöslich und in den oben genannten Reaktionslösungsmitteln nur mäßig löslich. Wird wie oben beispielhaft beschrieben aufgearbeitet, so lassen sie sich deshalb außer durch konventionelle Extraktion, bevorzugt mit Methylenchlorid, häufig auch durch einfaches Absaugen isolieren, wobei man jedoch eine geringe bis deutliche Ausbeuteminderung in Kauf nehmen muß. In den bisher untersuchten Beispielen waren die rohen Diphosphine der Formel la in Lösung stärker luftempfindlich als die meso-Diphosphine der Formel II, obwohl die gereinigten Verbindungen der Formel la als Feststoffe kaum und in Lösung nur mäßig luftempfindlich waren.

Wie man den Beispielen entnimmt, erhält man beispielsweise, wenn man vom Edukt der Formel Ib ausgeht, in der R¹ für Phenyl, die beiden Reste R² zusammen für ein doppelt gebundenes Sauerstoffatom, R³ für Isopropyl und X für Wasserstoff steht und das 98.5 % ee aufweist, mit 2.1 Äquivalenten n-Butyllithium und 2.2 Äquivalenten (-)-Spartein in Ether bei -70 bis -78 °C bei einer Lithiierungszeit von 30 Min. und bei Verwendung von Chlordiphenylphosphin, bei praktisch quantitativem Umsatz des Edukts der Formel Ib ein Rohprodukt, das das C₂symmetrische Diphosphin der Formel la und das meso-Diphosphin der Formel II im Verhältnis 97 : 3 enthält. Dieses Verhältnis wurde übereinstimmend nach verschiedenen Methoden bestimmt. Das Rohprodukt enthält >90 % d. Th. an Verbindung der Formel la. Setzt man als Edukt für den zweiten Lithiierungs-/Phosphinylierungs-Schritt kein optisch reines, sondern ein racemisches Monophosphin der Formel Ib ein, so erhält man bei Verwendung eines homochiralen Amins das optisch aktive C₂-symmetrische Diphosphin der Formel Ia im Gemisch mit dem entsprechenden meso-Diphosphin der Formel II. Geht man beim zweiten Lithiierungsschritt von racemischem Monophosphin der Formel Ib aus und verwendet ein achirales Amin, so erhält man racemisches Diphosphin der Formel la.

Zur Herstellung von optisch aktiven C₂-symmetrischen Verbindungen der Formel I, in der X für P(R¹)₂ steht, ist es auch möglich, beide ortho-Lithiierungs-/Phosphinylierungs-Schritte durchzuführen, ohne daß das zunächst entstehende Monophosphin zwischenisoliert wird, z. B. in einem Eintopfverfahren. Dieses Verfahren, das ebenfalls Gegenstand der vorliegenden Erfindung ist, ist dadurch gekennzeichnet, daß man eine Verbindung der Formel III, 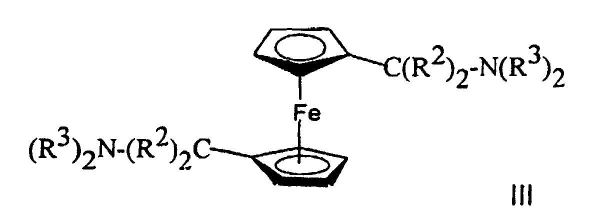 in der R² und R³ die oben angegebenen Bedeutungen haben, zunächst mit n-Butyllithium in Gegenwart eines homochiralen tertiären Amins, beispielsweise Spartein, deprotoniert, dann durch Zusatz eines Chlorphosphins der Formel CIP(R¹)₂, in der R¹ die oben angegebene Bedeutung hat und das bevorzugt in einem möglichst geringem Überschuß eingesetzt wird, phosphinyliert, dann ohne Isolierung des entstandenen Monophosphins durch erneuten Zusatz von n-Butyllithium den zweiten Deprotonierungsschritt ausführt und schließlich durch erneuten Zusatz des Chlorphosphins der Formel CIP(R¹)₂ die zweite Phosphinylierung bewirkt. Auch für dieses Verfahren gelten die oben zur Herstellung der Verbindungen der Formeln Ib und II gegebenen Erläuterungen, betreffend z. B. die Amine, die Lösungsmittel, die Mengen, die Reaktionstemperaturen oder gegebenenfalls zur Steigerung der Reinheit durchzuführende Umkristallisationen, entsprechend.

Gegenstand der vorliegenden Erfindung ist schließlich ein Verfahren zur Herstellung von racemischen Verbindungen der Formel I, in der die Reste R¹, R², R³ und X die oben angegebenen Bedeutungen haben, d. h. in der X im Falle der racemischen Monophosphine für Wasserstoff und im Falle der racemischen C₂symmetrischen Diphosphine für P(R¹)₂ steht, dadurch gekennzeichnet, daß man achirale Ferrocene der Formel III, 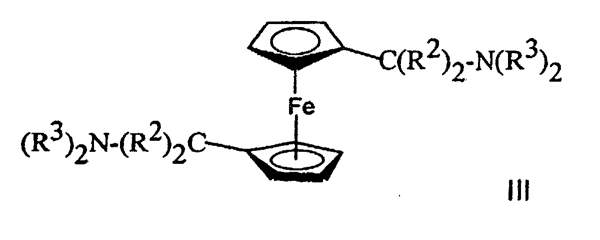 in der die Reste R² und R³ die oben angegebenen Bedeutungen haben, oder racemische Monophosphine der Formel I, in der X für Wasserstoff steht, mit n-Butyllithium in Gegenwart eines racemischen oder achiralen tertiären Amins, beispielsweise in Gegenwart von N,N,N',N'-Tetramethylethylendiamin (TMEDA), symmetrisch mono-ortho-lithiiert und die Monolithiumverbindung in situ mit einem Chlorphosphin der Formel ClP(R¹)₂ umsetzt, in der die Reste R¹ die oben angegebene Bedeutung haben, oder, im Falle der Herstellung der Diphosphine aus den Verbindungen der Formel III, schrittweise symmetrisch dilithiiert/diphosphinyliert. Auch für dieses Verfahren gelten die obigen Erläuterungen entsprechend.

Die Monophosphine und Diphosphine der Formeln I und II, in denen die Reste R² für Wasserstoff stehen, können nicht nur durch asymmetrische ortho-Lithiierung z. B. der N,N-disubstituierten 1,1'-Bis(aminomethyl)ferrocene der Formel III, in der die Reste R² für Wasserstoff stehen, hergestellt werden, sondern auch durch Reduktion der entsprechenden Bisamide der Formeln I und II, in denen die beiden Reste R² zusammen für ein doppelt gebundenes Sauerstoffatom stehen. Die Reduktion kann gemäß bekannten Verfahren mit verschiedenen Reduktionsmitteln erfolgen. Bevorzugt erfolgt sie mit dem Boran-Tetrahydrofuran-Komplex. Dabei werden zunächst die Boran-Addukte der Verbindungen der Formeln I und II, in der die Reste R² für Wasserstoff stehen, erhalten, aus denen dann in bekannter Weise die freien Phosphine bzw. die Übergangsmetallkatalysatoren hergestellt werden können (vgl. A.R. Muci, K.R. Campos, D.A. Evans, J. Am. Chem. Soc. 1995, 117, 9075).

Die erfindungsgemäßen Verbindungen der Formeln I und II eignen sich als Liganden für katalytisch aktive Übergangsmetallkomplexe. Die vorliegende Erfindung umfaßt daher auch die Verwendung von Verbindungen der Formeln I und II als Liganden bei der Herstellung von Übergangsmetallkomplexen bzw. die Verwendung der Verbindungen als Liganden in solchen Komplexen. Bevorzugt sind als Übergangsmetallelemente die Elemente Ruthenium, Rhodium, Iridium, Nickel, Palladium, Platin, Silber, Kupfer und Gold, besonders bevorzugt sind die Elemente Ruthenium, Rhodium, Iridium, Nickel, Palladium, Platin und Silber. Die Erfindung umfaßt insbesondere die Verwendung von chiralen bzw. optisch aktiven Verbindungen der Formel I als Liganden in chiralen Übergangsmetallkomplexen. Gemäß üblichen, in der Literatur beschriebenen Prozeduren, auf die hier insofern vollinhaltlich Bezug genommen wird, können die erfindungsgemäßen Verbindungen, insbesondere die optisch aktiven Phosphine der Formel I, in Analogie zu den Hayashi-Liganden der Formeln A, B und C zu isolierten Komplexen oder in situ-Komplexen der Übergangsmetalle umgesetzt werden, besonders bevorzugt zu Komplexen des Rutheniums, Rhodiums, Iridiums, Nickels, Palladiums, Platins oder Silbers (siehe T. Hayashi et al., Tetrahedron Lett. 1980, 21, 1871 und J. Am. Chem. Soc. 1982, 104, 180; W.R. Cullen et al., Organometallics 1985, 4, 346; Ferrocenes, A. Togni und T. Hayashi (Edit.), VCH, Weinheim, 1995; H.-U. Blaser, F. Spindler, ChimiaOggi 1995, Juni, Seite 11; N. C. Zanetti, F. Spindler, J. Spencer, A. Togni, G. Rihs, Organometallics 1996, 15, 860). Bevorzugt ist die Verwendung von optisch reinen oder weitgehend optisch reinen Verbindungen der Formel I für die Herstellung von optisch reinen oder weitgehend reinen Übergangsmetallkomplexen bzw. die Verwendung solcher Verbindungen als Liganden in solchen Komplexen. Die Erfindung umfaßt aber genauso die entsprechende Verwendung von racemischen Verbindungen der Formel I und achiralen Verbindungen der Formel II bei der Herstellung von racemischen oder achiralen Übergangsmetallkomplexen bzw. die Verwendung als Liganden in solchen Komplexen, wiederum bevorzugt Komplexen des Rutheniums, Rhodiums, Iridiums, Nickels, Palladiums, Platins, Silbers, Kupfers oder Golds, besonders bevorzugt Komplexen des Rutheniums, Rhodiums, Iridiums, Nickels, Palladiums, Platins oder Silbers.

Schließlich sind Gegenstand der Erfindung auch die Komplexe von Verbindungen der Formeln I und II, in denen die Substituenten R¹, R², R³ und X die oben angegebenen allgemeinen oder bevorzugten Bedeutungen haben, mit Übergangsmetallen, bevorzugt mit den Übergangsmetallen Ruthenium, Rhodium, Iridium, Nickel, Palladium, Platin, Silber, Kupfer und Gold, besonders bevorzugt mit den Übergangsmetallen Ruthenium, Rhodium, Iridium, Nickel, Palladium, Platin und Silber, sowie die Verwendung solcher Komplexe als Katalysatoren in katalytischen Reaktionen. Die erfindungsgemäßen Komplexe enthalten mindestens einen Liganden der Formeln I oder II (d. h., sie enthalten einen Liganden, der eine Verbindung der Formeln I oder II ist oder auch mehrere Liganden, die Verbindungen der Formeln I oder II sind). Sie können als weitere Liganden ein oder mehrere anorganische und/oder organische Ionen und/oder Moleküle enthalten. Die Auswahl weiterer Liganden, die in den Komplexen vorhanden sein können, richtet sich zum Beispiel nach dem vorgesehenen Verwendungszweck. Die Übergangsmetalle können in den Komplexen in ihren üblichen Wertigkeiten vorliegen. Die erfindungsgemäßen Übergangsmetallkomplexe sind, sowohl in isolierter Form als auch in situ erzeugt, wertvolle Katalysatoren für zahlreiche Reaktionen von organischen Verbindungen. Bevorzugt sind die Übergangsmetallkomplexe mit optisch reinen Liganden der Formel I, die als wertvolle Katalysatoren in asymmetrischen katalytischen Reaktionen verwendet werden können. Einige solcher Reaktionen sind beispielhaft in der folgenden Übersicht zusammengestellt:

Hydrierungen von prochiralen C=O-, C=C- und C=N-Gruppen;
Kupplungsreaktionen des Heck-Typs mit oder ohne Kohlenmonoxid-lnsertion; Kreuzkupplungen metallorganischer Verbindungen (z. B. Grignard-Verbindungen) mit organischen Halogeniden;
Hydrosilylierungen, Hydroborierungen und Hydroformylierungen von prochiralen C=O-, C=C- und C=N-Gruppen;
Nucleophile Substitutionen an Substraten, die in der allylischen Position eine nucleofuge Abgangsgruppe haben;
Isomerisierungen von Allylaminen zu den entsprechenden Enaminen.

Zusammenstellungen neuerer Literatur zu derartigen asymmetrischen Reaktionen finden sich in I. Ojima, Catalytic Asymmetric Synthesis, VCH, Weinheim, 1993 und in R. Noyori, Asymmetric Catalysis in Organic Synthesis, Wiley Interscience, 1994.

Die Erfindung umfaßt aber wiederum genauso die entsprechenden racemischen Übergangsmetallkomplexe und achirale Übergangsmetallkomplexe, bevorzugt der Elemente Ruthenium, Rhodium, Iridium, Nickel, Palladium, Platin, Silber, Kupfer und Gold, besonders bevorzugt der Elemente Ruthenium, Rhodium, Iridium, Nickel, Palladium, Platin und Silber, in denen racemische bzw. achirale Verbindungen der Formeln I oder II als Liganden enthalten sind, sowie die Verwendung dieser Komplexe als racemische Katalysatoren, insbesondere für die symmetrischen Varianten der in der obigen Übersicht zusammengestellten katalytischen Reaktionen.

### Beispiele

Allgemeines: Diethylether wurde frisch in einer Stickstoff-Atmosphäre von Natrium/Benzophenonketyl abdestilliert. (-)-Spartein wurde frisch im Vakuum durch eine kurze Vigreux-Kolonne von Calciumhydrid abdestilliert. Chlordiphenylphosphin wurde frisch im Hochvakuum (HV) durch eine kurze Vigreux-Kolonne destilliert. Alle verwendeten Lösungsmittel wurden vor dem Einbringen von Reagenzien mittels durchperlendem Argon von gelöstem Sauerstoff befreit. Bei Aufarbeitungen wurde mittels Durchleitens von Argon durch alle Lösungsmittel sowie zügiger Durchführung der Luftkontakt minimiert. Als kristalline Feststoffe sind alle hier beschriebenen Ferrocenverbindungen kaum luftempfindlich. In Lösung sind sie alle mehr oder weniger empfindlich, besonders die rohen C₂-symmetrischen Diphosphine der Formel la. NMR-spektroskopische Verschiebungen werden in ppm angegeben. Es bedeuten: Me Methyl, Et Ethyl, iPr Isopropyl, Ph Phenyl, Bu Butyl, BuLi Butyllithium.

### Herstellung der Ausgangsverbindung 1,1'-Bis(N,N-diisopropylamido)ferrocen (Formel III, (R²)₂: =O, R³: iPr); Synthesemethode 1

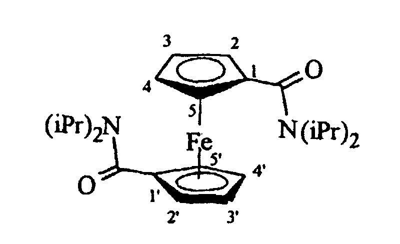

12.0 g (43.8 mmol) 1,1'-Ferrocendicarbonsäure (Aldrich) wurden in 63 ml Toluol suspendiert, die zuvor mittels eines durchperlenden Argonstroms entgast worden waren. Unter Argon-Atmosphäre wurden 15.2 ml (22.2 g, 174.9 mmol) Oxalylchlorid mit einer Spritze innerhalb 5 Min. zugegeben, gefolgt von 1.2 ml Dimethylformamid. Unter Aufschäumen und Gasentwicklung setzte eine heftige Reaktion ein, ohne daß sine nennenswerte Exothermie feststellbar war. Das Gemisch wurde 10 Min. bei Raumtemperatur gerührt. Das überschüssige Oxalylchlorid wurde am Rotationsverdampfer im Vakuum entfernt (Badtemperatur 30 °C), indem das Reaktionsgemisch auf 20 bis 30 ml Gesamtvolumen eingeengt wurde. Die noch gut rührbare Suspension wurde mit 250 ml Diethylether verdünnt und dann unter Argon auf 0 °C abgekühlt. Innerhalb 10 Min. wurden 32.2 ml (24.9 g, 245.9 mmol) Diisopropylamin zugetropft. Aufgrund der exothermen Reaktion stieg die Innentemperatur vorübergehend auf +5 °C. Die Farbe der Suspension änderte sich von dunkelorange nach hellgelb. Nach einstündigem Rühren bei 0 °C wurde eine 0.5 ml-Probe entnommen, mit 0.5 ml gesättigter wäßriger Ammoniumchloridlösung und 0.5 ml Diethylether versetzt und mit dem Eluenten Cyclohexan/Ethylacetat (1:1) durch Dünnschichtchromatographie (DC) analysiert, wobei vollständige Reaktion der Dicarbonsäure (R_{f} = 0.00) zur Titelverbindung (R_{f} = 0.37) und geringen Mengen eines etwas polareren Nebenprodukts (R_{f} = 0.25) festgestellt wurde. Nach weiterem einstündigen Rühren wurden unter Eiskühlung 200 ml gesättigte Ammoniumchlorid-Lösung und 50 ml Wasser (beides Argon-entgast) zugesetzt. Es wurde mit zweimal 200 ml Dichlormethan extrahiert, wobei während der Extraktion Argon durchgeperlt wurde. An der Phasengrenze setzte sich dabei ein schwarzer Mulm ab, der mit der Wasserphase abgetrennt wurde. Die vereinigten Extrakte wurden mit zweimal 250 ml Wasser, dann mit 250 ml gesättigter Kochsalzlösung gewaschen. Die organische Phase wurde 5 Min. über MgSO₄ getrocknet, filtriert, das Filtrat im Vakuum eingeengt und der Feststoff im Vakuum getrocknet. Man erhielt 17.2 g (39.0 mmol, 89 % Ausbeute) orangegelben Feststoff, der gemäß DC geringfügige polarere Verunreinigungen enthielt. Filtration durch Kieselgel mit Cyclohexan/Ethylacetat (3:1) ergab 15.0 g (34.1 mmol, 78 % Ausbeute) eines gelben, sehr voluminösen Feststoffs, Schmp. 136-137 °C (Lit.: P.J. Hammond et al., J. Organomet. Chem. 1986, 306, 367; Schmp. 127-128 °C).
¹H-NMR (200 MHz, CDCl₃): δ = 4.59 (t, J = 2 Hz, 4H, 2-,2'-,5-,5'-H), 4.43 (br s, 2H, CHMe₂), 4.38 (t, J = 2 Hz, 4H, 3-,3'-,4-,4'-H), 3.43 (br s, 2H, CHMe₂), 1.47 (br s, 12H, CH(CH₃)₂), 1.20 (br s, 12 H, CH(CH₃)₂).
¹³C-NMR (75.43 MHz, CDCl₃, Protonen-breitbandentkoppelt; Multiplizität bestimmt mit DEPT 135°): δ = 168.80 (2C, C=O), 83.23 (2C, 4°-C, C-1,-1'), 71.36 (4C, CH, C-2,-2',-5,-5'). 70.95 (4C, CH, C-3,-3',-4,-4'), 49.85 (2C, br, CH, CHMe₂), 46.19 (2C, br, CH, CHMe₂), 21.09 (8C, CH₃).
IR (KBr): v = 2968, 1633 (C=O), 1619 (C=C von Aryl), 1465, 1317 cm⁻¹.
MS (FAB, NBA): m/z (%) = 441 (55) [M+H⁺], 440 (100) [M⁺], 340 (22) [M+H⁺-HN(iPr)₂], 248 (15) [C₁₂H₁₈FeNO⁺].

### Herstellung der Ausgangsverbindung 1,1'-Bis(N,N-diisopropylamido)ferrocen (Formel III, (R²)₂: =O, R³: iPr); Synthesemethode 2

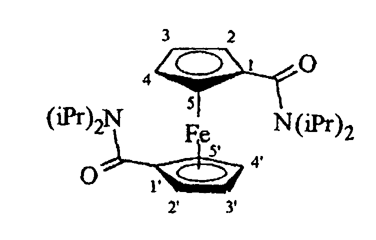

Zur Lösung von 10.0 g (36.5 mmol) 1,1'-Ferrocendicarbonsäure in 200 ml entgastem Toluol wurden bei Raumtemperatur unter Argon 10.0 ml (14.55 g, 114.6 mmol) Oxalylchlorid, sofort gefolgt von 1.0 ml (944 mg, 12.9 mmol) Dimethylformamid, gegeben. Unter Aufschäumen und starker Gasentwicklung, jedoch ohne nennenswerte Exothermie setzte die Reaktion ein. Die Innentemperatur stieg ohne Kühlung auf maximal 24 °C an. Man rührte 10 Min. bei Raumtemperatur und entfernte dann das überschüssige Oxalylchlorid, indem man im Vakuum auf ein Viertel des ursprünglichen Volumens einengte. Die aufkonzentrierte Lösung wurde mit 250 ml Toluol verdünnt und im Eisbad unter durchperlendem Argon auf 0 °C abgekühlt. Dann wurden nacheinander 11.2 ml (8.13 g, 80.3 mmol) entgastes Triethylamin und 9.6 ml (7.41 g, 73.2 mmol) entgastes Diisopropylamin zugetropft. Die dunkelrotbraune, klare Lösung wurde 1 Std. bei 0 °C, dann 16 Std. bei Raumtemperatur gerührt. Es wurden weitere 4.8 ml (3.70 g, 36.6 mmol) entgastes Diisopropylamin zugetropft und das Gemisch weitere 18 Std. bei Raumtemperatur gerührt. Aufarbeitung wie bei der Synthesemethode 1 beschrieben ergab 14.3 g (32.5 mmol, Rohausbeute 89 % d.Th.) orangebraunen Feststoff, der das gleiche polarere Nebenprodukt enthielt wie das nach der Synthesemethode 1 erhaltene Produkt. Filtration durch 300 g Kieselgel mit Cyclohexan/Ethylacetat (3:1) ergab 9.9 g (22.5 mmol, 62 % Ausbeute) der Titelverbindung als gelben Feststoff, Schmp. 134-135 °C, Zers. bei 258-262 °C unter Schwarzfärbung und Trübung der Schmelze.

### Beispiel 1

### 1-(Diphenylphosphino)-1',2-bis(N,N-diisopropylamido)ferrocen (Formel Ib, R¹: Ph, (R²)₂: =O, R³: iPr); Herstellung unter Verwendung von 4.40 Äquivalenten n-BuLi und 4.45 Äquivalenten (-)-Spartein in Diethylether

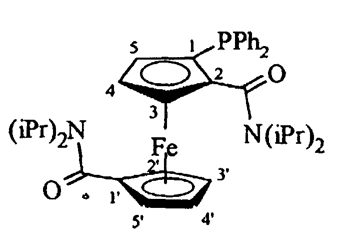

Zur Lösung von 7.30 g (7.16 ml, 31.15 mmol) (-)-Spartein in 130 ml Diethylether wurden bei -78 °C unter Argon innerhalb 5 Min. 19.25 ml (30.8 mmol) einer 1.6-molaren Lösung von n-Butyllithium in Hexan getropft. Die zunächst klare, gelbliche Lösung ging nach ca. 5 Min. in eine weiße Suspension über. Es wurde weitere 15 Min. bei -78 °C gerührt, dann die Lösung von 3.08 g (7.0 mmol) 1,1'-Bis(N,N-diisopropylamido)ferrocen in 35 ml Diethylether innerhalb 10 Min. zugetropft. Die tiefgelbe Suspension wurde 1 Stunde bei -78 °C unter Argon gerührt, dann wurden 7.5 ml (9.27 g, 42.0 mmol) Chlordiphenylphosphin mit einer Spritze innerhalb 5 Min. zugetropft. Innerhalb weiterer 5 Min. entstand aus der gelben Suspension eine braune, klare Lösung, die 30 Min. bei -78 °C gerührt wurde. Es wurde eine Probe (0.5 ml) entnommen, diese mit gesättigter Ammoniumchlorid-Lösung (0.5 ml) und Et₂O (0.5 ml) versetzt und mit dem Eluenten Cyclohexan/Ethylacetat (3:1) per DC untersucht. Es wurde gefunden, daß das Edukt der Formel III (R_{f} = 0.20) vollständig unter Bildung eines Produkts (Titelverbindung; R_{f} = 0.34) abreagiert hatte. Die entsprechenden Diphosphine der Formeln II und/oder Ia (R_{f} = 0.52, ungetrennt voneinander) waren nur in Spuren nachweisbar. 30 Min. nach der Probenentnahme (1 Std. nach der Zugabe des Chlorphosphins) wurde der Ansatz auf +20 °C erwärmt, dann wurden 160 ml einer gesättigten wäßrigen Ammoniumchlorid-Lösung innerhalb 5 Min. zugetropft. Das Gemisch wurde 15 Min. gerührt, dann wurden die Phasen getrennt. Die wäßrige Phase wurde mit 2 x 100 ml Ether extrahiert, die vereinigten Etherphasen wurden mit 200 ml Wasser, dann mit 200 ml gesättigter Kochsalzlösung gewaschen, über MgSO₄ getrocknet, filtriert und das Filtrat wurde im Vakuum eingeengt. Ein ³¹P-NMR-Spektrum des Rohprodukts (CDCl₃) zeigte die Resonanz der Titelverbindung der Formel Ib (δ = -22.92) und die Resonanz des entsprechenden C₂-symmetrischen Diphosphins der Formel la (δ = -22.00) im Verhältnis 95:5. Das entsprechende meso-Diphosphin der Formel II (δ = -22.38) war nicht sichtbar (< 0.5 %). Der Rückstand (10.4 g) wurde durch Flash-Chromatographie gereinigt (200 g Kieselgel 60 Å, 35-70 µm, Eluent Cyclohexan/Ethylacetat (3:1), 1.25 bar Stickstoff) und ergab 3.95 g (6.32 mmol, 90 % d. Th.) eines gelben Feststoffs, der gemäß Chiralphasen-HPLC-Analyse 80.0 % ee hatte (250 x 4.6 mm CSP Chiralpak AD; Eluent: n-Hexan/EtOH (20:1) plus 0.1 % Diethylamin, Fluß 1.0 ml/Min., 40 °C, Det. 248 nm; tᵣₑₜ (%): (+)-Titelverbindung der Formel Ib 6.21 Min. (90.0 %), (-)-Titelverbindung der Formel Ib 4.60 Min. (10.0 %)). Umkristallisation aus 30 ml heißem, Argon-entgasten n-Heptan ergab 2.52 g (4.03 mmol, 58 % d.Th.) gelbe Kristalle, Schmp. 146-148 °C (Zers.), [α]_{D}²⁰ = +235.8° (c = 1.013 in CH₂Cl₂), die gemäß Chiralphasen-HPLC-Analyse 98.5 % ee des (+)-Enantiomers der Titelverbindung aufwiesen.
¹H-NMR (300 MHz, CDCl₃): δ = 7.53 (m, 2H, p-H von C₆H₅), 7.16-7.40 (m, 8H, o-und m-H von C₆H₅), 4.80 (dt, J_{d} = 2.5 Hz, Jₜ = 1.3 Hz, 1H, 2'-H), 4.65 (td, Jₜ = 2.5 Hz, J_{d} = 1.4 Hz, 1H, 3'-H), 4.52 (dt, J_{d} = 2.5 Hz, Jₜ = 1.3 Hz, 1H, 5'-H), 4.45 (t, J = 2.5 Hz, 1H, 4-H), 4.38 (br s, 1H, CHMe₂), 4.36 (dt, J_{d} = 2.5 Hz, Jₜ = 1.2 Hz, 1H, 3-H), 4.25 (td, Jₜ = 2.5 Hz, J_{d} = 1.2 Hz, 1H, 4'-H), 3.91 (m, 1H, 5-H), 3.87 (br s, 1H, CHMe₂), 3.38 (br s, 1H, CHMe₂), 3.18 (br s, 1H, CHMe₂), 0.90-1.57 (m, 21H, 7 x CH₃), 0.50 (m, 3H, CH₃). Die Zuordnung der ¹H-Resonanzen zu den sieben Protonen der beiden Ferrocenringe beruht auf der Annahme, daß die Verbindung überwiegend in der Konformation vorliegt, die in der Formel im Beispiel 1 dargestellt ist, auf Überlegungen zur relativen Beeinflussung der Protonen in dieser Molekül-Konformation durch Anisotropien der beiden Carbonylgruppen und der Phenylringe, sowie auf einer Interpretation der Größe der Kopplungskonstanten. Es ist nicht völlig auszuschließen, daß die Zuordnungen der im folgenden genannten Protonenpaare gatauscht werden müssen: 2'-H mit 5'-H; 3'-H mit 4'-H; 3-H mit 4-H. ¹³C-NMR (75.43 MHz, CDCl₃, Protonen-breitbandentkoppelt): δ = 168.84 (1C, C=O), 166.77 (1C, C=O), 139.37 (d, ¹J_{C,P} = 13.8 Hz, 1C, direkt an Phosphor gebundenes C-Atom von C₆H₅), 138.05 (d, ¹J_{C,P} = 13.8 Hz, 1C, direkt an Phosphor gebundenes C-Atom von C₆H₅), 134.40 (d, ²J_{C,P} = 21.2 Hz, 1C, ortho-C von C₆H₅), 133.24 (d, ²J_{C,P} = 21.2 Hz, 1C, ortho'-C von C₆H₅), 128.76 (1C, para-C von C₆H₅), 128.24 (1C, para'-C von C₆H₅), 128.14 (d, J_{C,P} = 7.5 Hz, 2C, meta- und meta'-C von C₆H₅), 91.76 (d, ²J_{C,P} = 21.7 Hz, 1C, C-2), 82.90 (1C, C-1'), 80.48 (d, ¹J_{C,P} = 14.3 Hz, 1C, C-1), 74.71 (1C, C-5), 73.41 (1C, C-4'), 73.34 (d, ³J_{C,P} = 3.2 Hz, 1C, C-4), 72.15 (d, ³J_{C,P} = 4.2 Hz, 1C, C-3), 71.85, 71.75 und 71.36 (jeweils 1C, C-3',-2' und
-5'), 50.01 (br, 2C, NCH), 45.88 (br, 2C, NCH), 21.15 und 20.34 (zusammen 8C, CH₃).
³¹P-NMR (121.43 MHz, CDCl₃): δ = -22.92.
IR (KBr): v = 2966, 1623 (C=O), 1320, 701 cm⁻¹.
MS (FAB, NBA): m/z (%) = 625 (100) [M + H⁺], 624 (78) [M⁺], 581 (33) [M⁺-CHMe₂], 539 (48) ["581" - CH₂=CHMe].
UV (c = 13.52 µg/ml in Cyclohexan): λₘₐₓ (e) = 220 nm (38480 l/mol·cm), 250 nm (12330), 440 nm (323).
Berechnet für C₃₆H₄₅FeN₂O₂P (624.59): C 69.23, H 7.26, Fe 8.94, N 4.49, P 4.96; gefunden: C 69.4, H 7.0, Fe 8.7, N 4.3, P 5.2.

Die Absolutkonfiguration der Titelverbindung der Formel Ib entspricht der in der Formel im Beispiel 1 dargestellten Konfiguration. Sie ergibt sich eindeutig aus der Einkristall-Röntgenstrukturanalyse der entsprechenden Verbindung der Formel la, die mit dem Produkt des Beispiels 4 durchgeführt wurde.

### Beispiel 2

### 1-(Diphenylphosphino)-1',2-bis(N,N-diisopropylamido)ferrocen (Formel Ib, R¹: Ph, (R²)₂: =O, R³: iPr); Herstellung unter Verwendung von 1.9 Äquivalenten n-BuLi und 2.0 Äquivalenten (-)-Spartein in Diethylether

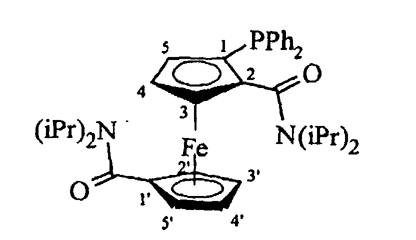

Zur Lösung von 8.52 g (8.35 ml, 36.35 mmol) (-)-Spartein in 100 ml Diethylether wurden bei -78 °C unter Argon innerhalb 5 Min. per Spritze 21.6 ml (34.5 mmol) einer 1.6-molaren Lösung von n-Butyllithium in Hexan getropft. Die milchig-trübe Suspension wurde 20 Min. bei -78 °C gerührt, dann wurde per Flex-Needle eine Lösung von 8.00 g (18.16 mmol) 1,1'-Bis(N,N-diisopropylamido)ferrocen in 250 ml Diethylether innerhalb 15 Min. zugetropft, so daß die Innentemperatur nicht über -74 °C anstieg. Die dunkelorangefarbene Lösung wurde 1 Stunde bei -78 °C unter Argon gerührt, dann wurden 9.80 ml (12.03 g, 54.5 mmol) Chlordiphenylphosphin per Spritze innerhalb 10 Min. zugetropft. Nach 10 Min. Reaktionszeit wurde eine DC-Probe entnommen, mit 0.5 ml Ether und 0.4 ml gesättigter Ammoniumchlorid-Lösung versetzt und mit dem Laufmittel Cyclohexan/Ethylacetat (3:1) analysiert, wobei praktisch vollständiger Umsatz angezeigt wurde. Nach insgesant 30 Min. Reaktionszeit wurde der Ansatz auf +4 °C erwärmt und es wurden 200 ml gesättigte Ammoniumchlorid-Lösung zugetropft. Es wurde 15 Min. gerührt, dann wurden die Phasen getrennt. Auch im Scheidetrichter wurde ständig Argon durch die Flüssigkeit geperlt. Bei den folgenden Extraktionen wurde die Phasendurchmischung durch die Stärke des durchgeleiteten Argonstroms geregelt. Die wäßrige Phase wurde auf diese Weise mit 2 x 100 ml Ether extrahiert. Die vereinigten etherischen Extrakte wurden auf die gleiche Weise mit 200 ml gesättigter Kochsalzlösung und dann 200 ml Wasser gewaschen. Dann wurde im Vakuum zur Trockne eingeengt und der Rückstand im HV getrocknet. Man erhielt 16.5 g orangebraunen, zähen Feststoff, der gemäß Chiralphasen-HPLC-Analyse Produkt von 80 % ee enthielt. Das Rohprodukt wurde in 200 ml entgastem Diethylether suspendiert und per Flex-Needle auf eine Kieselgelsäule (100 g Kieselgel 60 Å, 35-70 µm) aufgetragen. Die Suspension wurde mit Argon in die Säule gedrückt. Dann wurde mit 500 ml entgastem Diethylether nachgespült. Das Produkt lief mit der Front und wurde, zusammen mit unpolareren Verunreinigungen, gesammelt. Das Lösungsmittel wurde sofort im Vakuum abgedampft und der Rückstand im HV getrocknet. Man erhielt 14.8 g orangebraunen Feststoff. Dieser wurde unter Durchleiten von Argon in 300 ml n-Heptan durch zügiges Erhitzen zum Rückfluß gelöst. Man ließ unter fortdauerndem Durchleiten von Argon auf Raumtemperatur abkühlen und erhielt 6.56 g (10.5 mmol, 58 % d. Th.) eines orangegelben Pulvers, das gemäß Chiralphasen-HPLC-Analyse 98 % ee hatte.

### Beispiel 3

### 1-(Diphenylphosphino)-1',2-bis(N,N-diisopropylamido)ferrocen (Formel Ib, R¹: Ph, (R²)₂: =O, R³: iPr); Herstellung unter Verwendung von 1.9 Äquivalenten n-BuLi und 2.0 Äquivalenten (-)-Spartein in Toluol

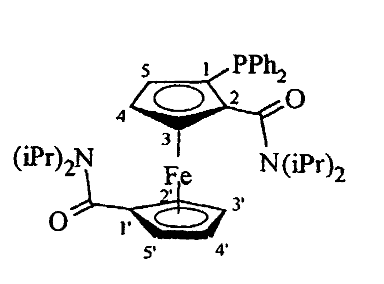

Zur Lösung von 21.3 g (90.9 mmol) (-)-Spartein in 40 ml Toluol (getrocknet über 4 Å-Molekularsieb) wurden bei -73 bis -78 °C unter Argon 54 ml (86.3 mmol) einer 1.6-molaren Lösung von n-Butyllithium in Hexan getropft. Die Suspension wurde 20 Min. bei -78 °C gerührt. Dann wurde eine Lösung von 20.0 g (45.4 mmol) 1,1'-Bis(N,N-diisopropylamido)ferrocen in 140 ml Toluol so zugetropft, daß die Reaktionstemperatur nicht über -73 °C anstieg. Es wurde 45 Min. bei -78 °C gerührt, dann wurden 24 ml (133.7 mmol) Chlordiphenylphosphin so zugetropft, daß die Innentemperatur nicht über -75 °C anstieg. Ein DC nach 10 Min. Reaktionszeit zeigte praktisch vollständigen Umsatz zum Monophosphin an. Nach 30 Min. Reaktionszeit wurde die Lösung auf +4 °C erwärmt und es wurden 350 ml gesättigte wäßrige Ammoniumchlorid-Lösung zugetropft. Die Phasen wurden 10 Min. verrührt, dann wurde die organische Phase abgetrennt. Die wäßrige Phase wurde unter Durchleiten von Argon mit 2 x 250 ml Toluol extrahiert. Die vereinigten Toluolphasen wurden mit 400 ml Wasser gewaschen und im Vakuum zur Trockne eingeengt. Das erhaltene Rohprodukt hatte gemäß Chiralphasen-HPLC 91 % ee. Es wurde in 450 ml Et₂O/Toluol (1:1) suspendiert, mit Argon in eine Säule aus 270 g Kieselgel (35-70 µm) gedrückt und mit 2 l Et₂O eluiert. Das Eluat wurde zur Trockne eingeengt und der Rückstand im Hochvakuum getrocknet. Der orangebraune Feststoff wurde unter Durchleiten von Argon aus 1.2 l heißem n-Heptan umkristallisiert. Man erhielt 21.3 g (34.1 mmol, 75 % d.Th.) orangegelbes Pulver von 99 % ee.

### Beispiel 4

### C₂-symmetrisches 1,1'-Bis(diphenylphosphino)-2,2'-bis(N,N-diisopropylamido)-ferrocen (Formel la, R¹: Ph, (R²)₂: =O, R³: iPr); Herstellung aus dem entsprechenden Monophosphin der Formel Ib unter Verwendung von 2.1 Äquivalenten n-BuLi und 2.2 Äquivalenten (-)-Spartein

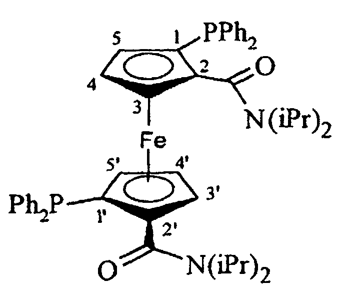

5.25 ml (8.4 mmol) einer 1.6-molaren Lösung von n-Butyllithium in Hexan wurden innerhalb 5 Min. bei -78 °C unter Argon zu der entgasten Lösung von 2.06 g (8.8 mmol) (-)-Spartein in 70 ml Diethylether getropft. Die Innentemperatur stieg dabei auf maximal -74 °C. Die klare, blaß-gelbe Lösung wurde 15 Min. bei -78 °C gerührt, dann wurde die Lösung von 2.50 g (4.0 mmol) (+)-1-(Diphenylphosphino)-1',2-bis(N,N-diisopropylamido)ferrocen (Beispiel 1; 98.5 % ee) in 30 ml entgastem Diethylether bei -78 °C innerhalb 5 Min. zugetropft. Die Innentemperatur stieg in der schwach exothermen Reaktion auf maximal -74 °C. Die orangefarbige Suspension wurde 30 Min. bei -78 °C gerührt, dann wurden 2.15 ml (2.65 g, 12.0 mmol) Chlordiphenylphosphin mit einer Spritze innerhalb 5 Min. zugegeben. Durch die exotherme Reaktion stieg die Innentemperatur auf maximal -71 °C. Das Gemisch wurde bei -78 °C nachgerührt. 15 Min. und 30 Min. nach der Chlorphosphinzugabe wurden Proben (je 0.2 ml) entnommen, die mit gesättigter Ammoniumchlorid-Lösung (je 0.2 ml) und CH₂Cl₂ (je 0.2 ml) versetzt wurden. DC-Analyse mit dem Laufmittel Toluol/Ethylacetat (15:1) zeigte bei der ersten Probe weitgehenden, bei der zweiten Probe vollständigen Umsatz des Edukts der Formel Ib (R_{f} = 0.06) zur Titelverbindung der Formel la (R_{f} = 0.20) und nur Spuren des meso-Diphosphins der Formel II (R_{f} = 0.24) an. 50 Min. nach der Chlorphosphin-Zugabe wurde der Ansatz auf Raumtemperatur erwärmt, mit 100 ml gesättigter wäßriger Ammoniumchlorid-Lösung versetzt und mit 2 x 100 ml Dichlormethan extrahiert. Die Extrakte wurden sofort im Vakuum eingeengt. Vom Rückstand wurde eine 100 mg-Probe für sofortige HPLC-, ¹H- und ³¹P-NMR-Analyse des Rohprodukts entnommen, der Rest sofort durch eine vorbereitete Säule (100 g Kieselgel) mit dem Laufmittel Toluol/Ethylacetat unter 1.3 bar Stickstoff flash-chromatographiert. Man erhielt 1.79 g (2.21 mmol, 55 % d.Th.) gelbes Pulver, Schmp. (unter Argon in abgeschmolzener Kapillare) 228-230 °C (Zers.), [α]_{D}²⁰ = +277.6° (c = 0.76 in CH₂Cl₂), das gemäß Chiralphasen-HPLC-Analyse ≥99 % ee hatte (250 x 4.6 mm CSP Chiralpak AD; Eluent: n-Hexan/iPrOH (87:13), Fluß 1.0 ml/Min., 40 °C, Det. 233 nm; tᵣₑₜ (+)-Titelverbindung der Formel la 5.47 Min.).
¹H-NMR (300 MHz, CDCl₃): δ = 7.15-7.37 (m, 20H, 4 x C₆H₅), 4.80 (dt, J_{d} = 2.5 Hz, Jₜ = 1.2 Hz, 2H, 3-,3'-H), 4.71 (t, J = 2.5 Hz, 2H, 4-,4'-H), 4.02 (br sept, J ca. 6 Hz, 2H, CHMe₂), 3.55 (m, 2H, 5-,5'-H), 3.18 (br sept, J ca. 6 Hz, 2H, CHMe₂), 1.41 (br d, J ca. 6 Hz, 6H, 2 x CH₃), 1.08 (m, 12H, 4 x CH₃), 0.57 (br d, J ca. 6 Hz, 6H, 2 x CH₃).
¹³C-NMR (75.43 MHz, CDCl₃, Protonen-breitbandentkoppelt): δ = 167.09 (2C, C=O), 139.17 (d, ¹J_{C,P} = 14.3 Hz, 2C, direkt an Phosphor gebundenes C-Atom von C₆H₅), 138.06 (d, ¹J_{C,P} = 14.8 Hz, 2C, direkt an Phosphor gebundenes C-Atom von C₆H₅), 133.80 (d, ²J_{C,P} = 20.7 Hz, 4C, o-C von C₆H₅), 133.37 (d, ²J_{C,P} = 21.2 Hz, 4C, o'-C von C₆H₅), 128.44 (2C, p-C von C₆H₅), 128.26 (2C, p'-C von C₆H₅), 128.10 (d, ³J_{C,P} = 6.9 Hz, 4C, m-C von C₆H₅), 128.04 (d, ³J_{C,P} = 6.3 Hz, 4C, m'-C von C₆H₅), 90.48 (d, ²J_{C,P} = 18.0 Hz, 2C, C-2,2'), 81.32 (d, ¹J_{C,P} = 14.3 Hz, 2C, C-1,1'), 76.77 (2C, C-3,3'), 74.93 (d, ³J_{C,P} = 6.4 Hz, 2C, C-4,4'), 72.70 (d, ²J_{C,P} = 3.7 Hz, 2C, C-5,5'), 50.11 (2C, NCH), 45.88 (2C, NCH), 20.42 (8C, CH₃).
³¹P-NMR (121.43 MHz, CDCl₃): δ = -22.00.
IR (KBr): v = 2964, 1630 (C=O), 1444, 1328, 697 cm⁻¹.
MS (FAB, NBA): m/z (%) = 809 (100) [M + H⁺], 808 (97) [M⁺], 765 (41) [M⁺ - iPr], 731 (14), 723 (12) ["765" - CH₂=CHMe].
UV (c = 12.00 µg/ml in Cyclohexan): λₘₐₓ(ε) = 223 nm (52760 l/mol·cm), 260 nm (14290), 445 nm (196).
Berechnet für C₄₈H₅₄FeP₂N₂O₂ (808.77): C 71.29, H 6.73, N 3.46, P 7.66, Fe 6.91; gefunden: C 70.8, H 6.5, N 3.2, P 7.4, Fe 6.5.

Einkristalle der Titelverbindung für eine Röntgenstrukturanalyse wurden analog zum Beispiel 7 nach der Verdunstungsmethode (CH₂Cl₂/Methyl-tert-Butylether) gewonnen. Die kristallographischen Daten wurden beim Cambridge Crystallographic Data Center, 12 Union Road, Cambridge CB2 1EZ hinterlegt. Die Röntgenstrukturanalyse bestätigt die Struktur. Die ermittelte Absolutkonfiguration der Titelverbindung der Formel la entspricht der in der Formel im Beispiel 4 dargestellten Konfiguration. Sie ergibt sich eindeutig aus der Anwendung der Bijvoet-Methode (J.M. Bijvoet, A.F. Peerdeman, A.J. van Bommel, Nature (London) 1951, 168, 271) auf die Einkristall-Röntgenstrukturanalysedaten. Man entnimmt der Röntgenstruktur, daß die beiden Ferrocenringe in der C₂-symmetrischen Titelverbindung der Formel la nahezu ekliptisch zueinander angeordnet sind. Im Gegensatz dazu sind die Ferrocenringe in Kristallen der entsprechenden meso-Verbindung (Beispiel 7) ideal versetzt zueinander ("staggered") angeordnet.

Die HPLC-Analyse (250 x 4.6 mm CSP Chiralpak AD; Eluent: n-Hexan/iPrOH (87:13), Fluß 1.0 ml/Min., 40 °C, Det. 233 nm; tᵣₑₜ (+)-Titelverbindung der Formel la 5.47 Min., entsprechende meso-Verbindung der Formel II (Beispiel 7) 8.70 Min.) der vor der Flash-Chromatographie entnommenen Probe des Rohprodukts zeigte, daß das Verhältnis der Verbindungen la zu II 97:3 war. Die ³¹P-Resonanz der Verbindung der Formel la war gegenüber der der Verbindung der Formel II um Δδ = 0.38 ppm zu tieferem Feld verschoben. Im Rohprodukt betrug das Intensitätsverhältnis der ³¹P-Resonanzen der Verbindungen la und II 97.2 : 2.8. Auch aus dem ¹H-NMR des Rohprodukts wurde abgeschätzt, daß der Gehalt an meso-Verbindung der Formel II unter 5 % liegt. Da die Verbindung der Formel la deutlich rascher als die der Formel II zum Monophosphinoxid oxidiert wird, müssen diese Analysen sofort nach der Probenentnahme unter Verwendung entgaster Lösungsmittel durchgeführt werden. ³¹P-NMR-Spektren partiell oxidierter Proben der Titelverbindung der Formel la zeigen zwei diastereomere Monophosphinoxide exakt im Verhältnis 1:1 an, deren Ph₂P-Resonanzen bei δ = -20.81 bzw. -22.68 und deren Ph₂PO-Resonanzen bei δ = +37.50 bzw. +35.62 erscheinen.

### Beispiel 5

C₂-symmetrisches 1,1'-Bis(diphenylphosphino)-2,2'-bis(N,N-diisopropylamido)-ferrocen (Formel la, R¹: Ph, (R²)₂: =O, R³: iPr); Herstellung aus dem entsprechenden Monophosphin der Formel Ib unter Verwendung von 2.0 Äquivalenten n-BuLi und 2.1 Äquivalenten (-)-Spartein mit verbesserter Aufarbeitung und Reinigung des Produkts ohne Chromatographie

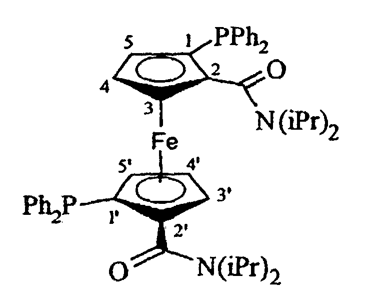

Zur Lösung von 5.92 g (5.80 ml, 25.2 mmol) (-)-Spartein in 30 ml Diethylether wurden per Spritze bei -78 °C 15.00 ml (24.0 mmol) einer 1.6-molaren Lösung von n-Butyllithium in Hexan getropft und das Gemisch wurde 20 Min. bei -78 °C gerührt. Dann wurde die Lösung von 7.46 g (11.94 mmol) (+)-1-(Diphenylphosphino)-1',2-bis(N,N-diisopropylamido)ferrocen (98 % ee) in 190 ml Diethylether per Flex-Needle so zugetropft, daß die Innentemperatur nicht über -77 °C anstieg. Es wurde 1 Std. bei -78 °C gerührt, dann wurden 4.00 ml Chlordiphenylphosphin per Spritze innerhalb 5 Min. zugetropft. Ein DC nach 10 Min. Reaktionszeit zeigte praktisch vollständigen Umsatz zum Diphosphin an. Nach 30 Min. wurde das Kältebad entfernt und die orangefarbene Suspension auf +3 °C erwärmt. Anschließend wurden 150 ml halbkonzentrierte wäßrige Ammoniumchlorid-Lösung zugetropft und das Zweiphasengemisch wurde 10 Min. intensiv verrührt. Dabei bildete sich bereits ein gelber Niederschlag. Zur vollständigeren Ausfällung des Produkts wurden 200 ml n-Pentan zugesetzt. Der Niederschlag wurde abgesaugt, mit n-Pentan gewaschen und im HV getrocknet. Erhalten wurden 6.23 g (7.70 mmol, 65 % d. Th.) gelbe Kristalle von 100 % ee und >99 % chemischer Reinheit (Chiralphasen-HPLC). Sofortiges Einengen der Mutterlauge im Vakuum und Verreiben des Rückstands mit n-Pentan ergab weitere 0.82 g, die aus 75 % der Titelverbindung der Formel la und 25 % des entsprechenden meso-Diphosphins der Formel II bestanden.

### Beispiel 6

C₂-symmetrisches 1,1'-Bis(diphenylphosphino)-2,2'-bis(N,N-diisopropylamido)-ferrocen (Formel la, R¹: Ph, (R²)₂: =O, R³: iPr); Herstellung aus dem Racemat des entsprechenden Monophosphins der Formel rac-Ib unter Verwendung von 2.1 Äquivalenten n-BuLi und 2.2 Äquivalenten (-)-Spartein und unter Abtrennung des entsprechenden meso-Diphosphins der Formel II

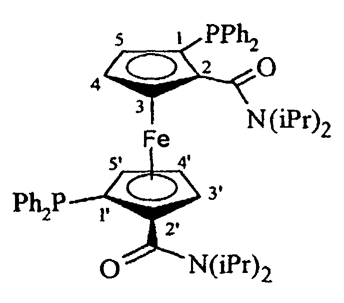

Racemisches 1-(Diphenylphosphino)-1',2-bis(N,N-diisopropylamido)ferrocen wurde durch Lithiierung von 1,1'-Bis(N,N-diisopropylamido)ferrocen mit 4.45 Äquivalenten N,N,N',N'-Tetramethylethylendiamin (TMEDA) und 4.40 Äquivalenten n-BuLi in Et₂O bei -78 °C, gefolgt von der Zugabe von 6.0 Äquivalenten Chlordiphenylphosphin und Aufarbeitung analog Beispiel 1 in 50 % Ausbeute hergestellt.

Zur Lösung von 3.0 g (12.8 mmol) (-)-Spartein in 84 ml Diethylether wurden bei -78 °C 7.6 ml (12.1 mmol) einer 1.6-molaren Lösung von n-Butyllithium in Hexan getropft. Nach 15 Min. wurde innerhalb 5 Min. bei -78 °C eine Lösung von 3.6 g (5.76 mmol) racemischem 1-(Diphenylphosphino)-1',2-bis(N,N-diisopropylamido)ferrocen in 60 ml Diethylether zugetropft. Die rotbraune klare Lösung wurde 1 Std. bei -78 °C gerührt, dann wurden 3.1 ml (3.82 g, 17.3 mmol) Chlordiphenylphosphin in 5 Min. zugetropft. Eine DC-Probe nach 30 Min. zeigte vollständigen Umsatz des Edukts an. Nach insgesamt 1 Std. Reaktionszeit wurde der Ansatz auf 20 °C erwärmt, es wurden 150 ml gesättigte Ammoniumchlorid-Lösung zugetropft und das Gemisch wurde 5 Min. kräftig durchgerührt. Die Aufarbeitung erfolgte wie im Beispiel 5 beschrieben. Das Rohprodukt (8.0 g brauner, amorpher Feststoff) enthielt das C₂-symmetrische Diphosphin der Formel la und das entsprechende meso-Diphosphin der Formel II im Verhältnis von ca. 1:1 (DC). Es wurde mit dem Laufmittel Toluol/Ethylacetat (15:1) durch 200 g Kieselgel (35-70 µm) flash-chromatographiert, wobei das unpolarere meso-Diphosphin der Formel II abgetrennt und die reine Titelverbindung der Formel la gesammelt wurde. Erhalten wurden 1.95 g (2.41 mmol, 40 % d. Th.) gelber Feststoff der Titelverbindung la von >99 % ee und 2.20 g (2.72 mmol, 46 % d. Th.) hellbrauner Feststoff, der gemäß Chiralphasen-HPLC-überwiegend aus dem entsprechenden meso-Diphosphin der Formel II und 30 % C₂-symmetrischem Diphosphin der Formel la von 99 % ee bestand.

### Beispiel 7

### meso-1,1'-Bis(diphenylphosphino)-2,2'-bis(N,N-diisopropylamido)ferrocen (Formel II, R¹: Ph, (R²)₂: =O, R³: iPr); Herstellung unter Verwendung von 4.40 Äquivalenten sec-BuLi und 4.45 Äquivalenten (-)-Spartein

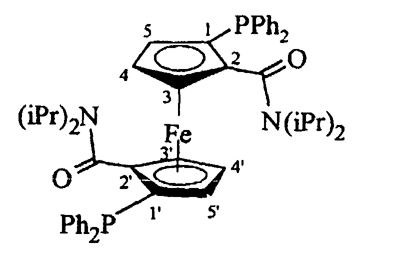

Zur Lösung von 3.56 g (3.5 ml, 15.17 mmol) (-)-Spartein in 65 ml Diethylether wurden bei -78 °C unter Argon innerhalb 10 Min. 11.5 ml (15.0 mmol) einer 1.3-molaren Lösung von sec-Butyllithium in Cyclohexan getropft. Es wurde 15 Min. bei -78 °C gerührt und dann die Lösung von 1.50 g 1,1'-Bis(N,N-diisopropylamido)ferrocen (3.41 mmol) in 27 ml Diethylether innerhalb 10 Min. zugetropft. Es wurde 2 Stunden bei -78 °C unter Argon gerührt. Mit einer Spritze wurden 3.7 ml (4.51 g, 20.4 mmol) Chlordiphenylphosphin innerhalb 10 Min. zugetropft. Die vorher braune, klare Lösung färbte sich dabei zunächst tiefbraun bis fast schwarz. Nach beendeter Zugabe ging diese dunkle Lösung in eine orange Suspension über. Nach 30 Min. Rühren bei -78 °C wurde eine DC-Probe (0.5 ml) entnommen, mit gesättigter Ammoniumchlorid-Lösung (0.5 ml) und Et₂O (0.5 ml) versetzt und mit dem Laufmittel Cyclohexan/Ethylacetat (3:1) per DC analysiert. Die Reaktion war praktisch vollständig (Produkt: R_{f} = 0.45). Das entsprechende Monophosphin der Formel Ib (vgl. Beispiel 1; R_{f} = 0.27) und das Edukt der Formel III (R_{f} = 0.13) waren nur in geringen Spuren nachweisbar. Der Ansatz wurde weitere 30 Min. gerührt und auf Raumtemperatur erwärmt. Dann wurden 80 ml gesättigte Ammoniumchlorid-Lösung innerhalb 5 Min. zugetropft und das Gemsich wurde weitere 15 Min. gerührt. Der gelbe Feststoff wurde aus der zweiphasigen Suspension abgesaugt, mit 10 ml Wasser verrührt und erneut abgesaugt, dann mit 10 ml Ether gewaschen und im HV getrocknet (1.9 g gelber Feststoff, im obigen DC-Laufmittel homogen; in Toluol/Ethylacetat (10:1) erkennt man neben dem Produkt (R_{f} = 0.33) ca. 4-5 % des Isomeren der Formel la (R_{f} = 0.29). Durch sofortige Extraktion des Filtrats mit 2 x 50 ml Methylenchlorid, Waschen der vereinigten Extrakte mit Wasser (50 ml) und Kochsalzlösung (50 ml), gefolgt vom Einengen im Vakuum und Verreiben des Rückstands mit Ether, wurden weitere 0.45 g gelber Feststoff erhalten, der gemäß DC und NMR ca. 90 %ig war bei einem unveränderten Anteil an Diphosphin der Formel la von 4-5 %. Gesamtausbeute:
2.35 g (2.91 mmol, 85 % d. Th.), Schmp. 223-224 °C (Zers.), [α]_{D}²⁰ = +4.3° (c = 0.74 in CH₂Cl₂).
¹H-NMR (300 MHz, CDCl₃): δ = 7.57 (m, 4H, p-H von C₆H₅), 7.10-7.37 (m, 16H, o-und m-H von C₆H₅), 4.74 (s, 2H, 3-,3'-H), 4.20 und 4.19 (2 x s, 4H, 4-,4'-,5-,5'-H), 3.79 (br s, 2H, CHMe₂), 3.11 (br s, 2H, CHMe₂), 1.25-0.70 (m, 18H, CH₃), 0.50 (br s, 6H, CH₃).
¹³C-NMR (75.43 MHz, CDCl₃, Protonen-breitbandentkoppelt): δ = 166.36 (2C, C=O), 139.78 (d, ¹J_{C,P} = 13.3 Hz, 2C, direkt an Phosphor gebundene C-Atome von C₆H₅), 138.25 (d, ¹J_{C,P} = 13.7 Hz, 2C, direkt an Phosphor gebundene C-Atome von C₆H₅), 134.75 (d, ²J_{C,P} = 22.8 Hz, 2C, ortho-C von C₆H₅), 132.98 (d, ²J_{C,P} = 20.6 Hz, 2C, ortho-C von C₆H₅), 128.89 (2C, para-C von C₆H₅), 128.14 (d, ³J_{C,P} = 7.8 Hz, 4C, meta-C von C₆H₅), 128.05 (2C, para-C von C₆H₅), 91.07 (d, ²J_{C,P} = 21.2 Hz, 2C, C-2,2'), 81.30 (d, ¹J_{C,P} = 13.7 Hz, 2C, C-1,1'), 75.41 (2C, C-5,5' oder C-4,4'), 74.77 (d, J_{C,P} = 4 Hz, 2C, C-4,4' oder C-5,5'), 72.86 (d, ³J_{C,P} = 4 Hz, 2C, C-3,3'), 49.94 (br, 2C, NCH), 45.78 (br, 2C, NCH), 20.36 (8C, CH₃).
³¹P-NMR (121.43 MHz, CDCl₃): δ = -22.38 (2 isochrone P-Atome).
IR (KBr): v = 3068 (Aryl-H), 2964, 1632 (C=O), 1455, 1335, 1283, 823, 740, 696 cm⁻¹.
MS (FAB, NBA/LiCl): m/z (%) = 815 (100) [M + Li⁺], 809 (70) [M + H⁺], 765 (40) [M⁺-iPr], 723 (15) ["765" - CH₂=CHMe].
UV (c = 14.84 µg/ml in Cyclohexan): λₘₐₓ (ε) = 223 nm (47960 l/mol·cm), 250 nm (16350), 445 nm (327).

Die HPLC-Analyse (CSP Chiralpak AD) zeigte ein Verhältnis der Verbindungen der Formeln II und la von 96.4 : 3.6 an. Die Röntgen-Pulverdiffraktion dieses Feststoffs zeigte ebenfalls Spuren der Verbindung der Formel la als Verunreinigung an. Isomerenfreie, analytisch reine Kristalle für eine Einkristall-Röntgenstrukturanalyse und die Elementaranalyse des meso-Diphosphins der Formel II wurden durch Umkristallisation aus Methyl-tert-Butylether/ Dichlormethan nach der Verdunstungsmethode gewonnen. Hierzu wurden 100 mg des obigen Produkts in 7 ml entgastem Methyl-tert-Butylether suspendiert und es wurde solange entgastes Dichlormethan zugetropft, bis eine klare Lösung vorlag. Diese wurde in einem offenen Kölbchen in einem großen, mit Argon gefüllten, geschlossenen Exsiccator 2 Tage im Dunkeln stehen gelassen. Durch Verdunstung des Dichlormethans trat Übersättigung der Lösung ein und es bildeten sich große Kristalle. Die Mutterlauge wurde abdekantiert und die Kristalle wurden mit frisch destilliertem Et₂O gewaschen. Die Probenanteile für Elementaranalyse, Drehwert und Schmelzpunkt wurde im HV scharf getrocknet. Die Kristalle für die Röntgenstrukturanalyse und die Wiederholung der Röntgen-Pulverdiffraktion wurden nicht getrocknet. Schmp. 229-231 °C (Zers.), [α]_{D}²⁰ = 0° (c = 1 in CH₂Cl₂); berechnet für C₄₈H₅₄FeP₂N₂O₂ (808.77): C 71.29, H 6.73, N 3.46, P 7.66, Fe 6.91; gefunden: C 70.9, H 6.6, N 3.3, P 7.4, Fe 6.3. Im Röntgen-Pulverdiffraktogramm und im DC war die isomere Verbindung der Formel Ia jetzt nicht mehr nachzuweisen. Die Einkristall-Röntgenstrukturanalyse bestätigt die Struktur der Titelverbindung. Die kristallographischen Daten wurden beim Cambridge Crystallographic Data Center, 12 Union Road, Cambridge CB2 1EZ hinterlegt. Die beiden Ferrocenringe sind ideal versetzt zueinander ("staggered") angeordnet. Die Verbindung liegt in der Konformation vor, die in der Formel im Beispiel 7 angegeben ist.

### Beispiel 8

### meso-1,1'-Bis(diphenylphosphino)-2,2'-bis(N,N-diisopropylamido)ferrocen (Formel II, R¹: Ph, (R²)₂: =O, R³: iPr); Herstellung unter Verwendung von 3.00 Äquivalenten sec-BuLi und 3.05 Äquivalenten (-)-Spartein

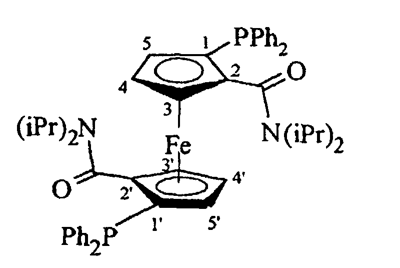

Ein Ansatz analog zu Beispiel 7 in Et₂O (65 ml/g 1,1'-Bis(N,N-diisopropylamido)ferrocen), aber mit verringerter Menge der chiralen Lithiumbase (3.00 Äquivalente sec-BuLi, 3.05 Äquivalente (-)-Spartein), ergab bei unveränderter Lithiierungsdauer (2.0 Std. bei -74 bis -78 °C) sowie bei verringerter Menge Chlordiphenylphosphin (4.00 Äquivalente) das Titelprodukt in einer Ausbeute von 76 % d.Th. Die Diastereoselektivität war unverändert gegenüber Beispiel 7.

### Beispiel 9

### meso-1,1'-Bis(diphenylphosphino)-2,2'-bis(N,N-diisopropylamido)ferrocen (Formel II, R¹: Ph, (R²)₂: =O, R³: iPr); Herstellung unter Verwendung von 3.00 Äquivalenten sec-BuLi und 3.05 Äquivalenten N,N,N',N'-Tetramethylethylendiamin (TMEDA)

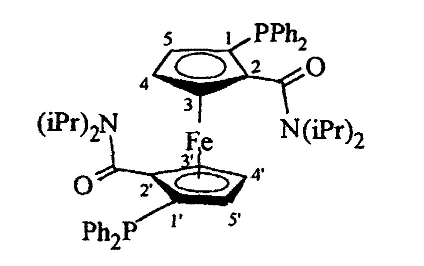

Ein Ansatz analog zu Beispiel 8, aber mit TMEDA anstatt (-)-Spartein, ergab das Titelprodukt in einer Ausbeute von 64 % d.Th. Die Diastereoselektivität war unverändert gegenüber den Beispielen 7 und 8.

### Beispiel 10

### Palladiumdichlorid-Komplex des C₂-symmetrischen 1,1'-Bis(diphenylphosphino)-2,2'-bis(N,N-diisopropylamido)ferrocens (Formel Ia · PdCl₂, R¹: Ph, (R²)₂: =O, R³: iPr); Herstellung aus dem entsprechenden homochiralen Diphosphin der Formel Ia und Bis(acetonitril)palladium(II)-chlorid ((CH₃CN)₂PdCl₂)

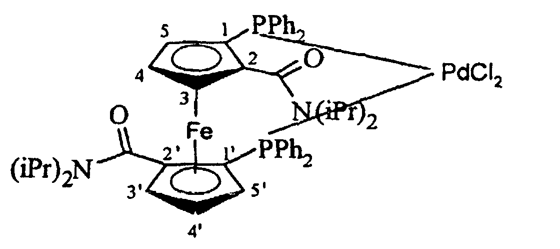

Zur Suspension von 195 mg (0.75 mmol) Bis(acetonitril)palladium(II)-chlorid (Fluka) in 8 ml entgastem trockenem Toluol und 6 ml entgastem trockenem Dichlormethan wurden bei Raumtemperatur (22 °C) in einer Portion 606 mg (0.75 mmol) kristallines, homochirales C₂-symmetrisches 1,1'-Bis(diphenylphosphino)-2,2'bis(N,N-diisopropylamido)ferrocen (aus Beispiel 5) gegeben. Zur Suspension wurde weiteres Dichlormethan (7 ml) gegeben, bis eine klare Lösung vorlag. Ein DC (Laufmittel Toluol/Ethylacetat (10:1)) nach 30 Min. zeigte eine vollständige Reaktion des Diphosphins der Formel la an. Der Lösung wurden 40 ml entgastes n-Pentan zugesetzt, der Niederschlag wurde abgesaugt und mit n-Pentan nachgewaschen. Nach Trocknen im HV wurden 660 mg (0.67 mmol, 89 % d. Th.) orangebraunes Pulver, Schmp. (unter Argon in abgeschmolzener Kapillare) 265 °C (Zers.; Schwarzfärbung ab 230 °C), [α]_{D}²⁰ = -484.0° (c = 0.505 in CH₂Cl₂) erhalten. Es enthielt gemäß ¹H-, ¹³C- und ³¹P-NMR außer Spuren Toluol keine Verunreinigungen und war 99.2 %ig gemäß HPLC-Analyse (125 x 4 mm SP Purospher 5 µm; Eluent CH₃CN/Wasser (40:30) plus 0.3 % KH₂PO₄ plus 0.1 % Camphersulfonsäure; Fluß 1.0 ml/Min.; 40 °C; Det. 216 nm; tᵣₑₜ der Titelverbindung 8.95 Min.).
¹H-NMR (300 MHz, CD₂Cl₂): δ = 8.61 (br s, 4H, arom. H), 8.15 (m, 4H, arom. H), 7.30-7.50 (m, 12H, arom. H), 4.35 (s, 2H, Ferrocenyl-H), 4.30 (s, 4H, Ferrocenyl-H), 3.92 (sept, J = 6.5 Hz, 2H, CHMe₂), 3.08 (sept, J = 6.5 Hz, 2H, CHMe₂), 1.07 (d, J ca. 6.5 Hz, 6H, 2 x CH₃), 1.03 (d, J ca. 6.5 Hz, 12H, 4 x CH₃), 0.76 (d, J = 6.5 Hz, 6H, 2 x CH₃).
¹³C-NMR (75.43 MHz, CD₂Cl₂, Protonen-breitbandentkoppelt; Multiplizität bestätigt mit DEPT 135°): δ = 166.99 (2C, C=O), 136.76 (d, ⁴J_{C,P} = 4.7 Hz, 2C, p-C von C₆H₅), 136.69 (d, ⁴J_{C,P} = 5.3 Hz, 2C, p'-C von C₆H₅), 135.49 (d, ¹J_{C,P} = 26.0 Hz, 2C, direkt an Phosphor gebundene C-Atome von C₆H₅), 134.81 (d, ¹J_{C,P} = 26.0 Hz, 2C, direkt an Phosphor gebundene C-Atome von C₆H₅), 130.15 (d, ²J_{C,P} = 19.1 Hz, 2C, o- und o'-C von C₆H₅), 127.52 (d, ³J_{C,P} = 5.8 Hz, 2C, m'-C von C₆H₅), 127.44 (d, ³J_{C,P} = 5.8 Hz, 4C, m-C von C₆H₅), 127.37 (d, ³J_{C,P} = 5.9 Hz, 2C, m'-C von C₆H₅), 98.92 (d, ¹J_{C,P} = 29.1 Hz, 2C, C-1,1'), 98.53 (d, ²J_{C,P} = 29.7 Hz, 2C, C-2,2'), 86.46 (2C, C-3,3'), 70.01 (2C, C-4,4'), 68.29 (2C, C-5,5'), 50.18 (2C, NCH), 46.76 (2C, NCH), 21.74 (4C, CH₃), 20.40 (2C, CH₃), 20.22 (2C, CH₃).
³¹P-NMR (121.43 MHz, CD₂Cl₂): δ = +50.03.
IR (KBr): v = 3054, 2967, 1612 (C=O), 1436, 1332, 693 cm⁻¹.
MS (FAB, NBA): m/z (%) = 990 (1), 989 (2), 988 (4), 987 (4), 986 (6), 985 (4), 984 (5), 983 (4), 982 (2) (Peakserie für [M+H⁺]); 956 (7), 955 (15), 954 (27), 953 (55), 952 (48), 951 (96), 950 (62), 949 (100), 948 (63), 947 (30), 946 (5) (Peakserie für M-CI; Intensitätsverhältnis in Übereinstimmung mit Isotopensimulation für C₄₈H₅₄FeP₂N₂O₂PdCl); 919 (4), 918 (7), 917 (8), 916 (14), 915 (10), 914 (19), 913 (13), 912 (6) (Peakserie für M-2Cl); 376 (66) (c-C₅H₃(PPh₂)(CONiPr₂)⁺); 292 (42). Zur Herstellung von Einkristallen für die Röntgenstrukturanalyse wurde eine Kristallisierschale zweimal mit je 5 ml Hexamethyldisilazan ausgespült, einmal mit Methanol nachgespült und an der Luft getrocknet. 40 mg des Komplexes wurden in der Kristallisierschale in 20 ml entgastem Dichlormethan gelöst und zusammen mit einer Kristallisierschale, die 10 ml entgastes n-Heptan enthielt, in einen Argongefüllten Exsiccator gestellt. Innerhalb von 2 Tagen bildeten sich Kristalle. Das Lösungsmittel wurde abdekantiert, die Kristalle wurden mit n-Heptan gewaschen, im Argon-Strom getrocknet und ein guter Kristall wurde in ein Mark-Röhrchen eingeschmolzen und vermessen. Die kristallographischen Daten wurden beim Cambridge Crystallographic Data Center, 12 Union Road, Cambridge CB2 1EZ hinterlegt. Das Ergebnis der Röntgenstrukturanalyse bestätigt die Struktur des Titelkomplexes. Man erkennt, daß die Koordinationssphäre um das Palladiumatom weitgehend quadratisch-planar ist. Die beiden Ferrocenylringe sind beinahe ekliptisch angeordnet und die beiden Phosphinogruppen stehen in der Aufsicht beinahe direkt hintereinander. Im Kristallgitter ist auch konformativ die C₂-Symmetrie gut erhalten. Es liegt ein Solvat vor, Dichlormethan ist in das Kristallgitter eingebaut.

### Beispiel 11

### 1,5-Cyclooctadien-rhodium(I)-tetrafluoroborat-Komplex des C₂-symmetrischen 1,1'-Bis(diphenylphosphino)-2,2'-bis(N,N-diisopropylamido)ferrocens (Formel [Ia · (COD)Rh(I)]⁺BF₄⁻, R¹: Ph, (R²)₂: =O, R³: iPr); Herstellung aus dem entsprechenden homochiralen Diphosphin der Formel la und Bis(1,5-cyclooctadien)rhodium(I)-tetrafluoroborat ([Rh(I)(COD)₂]⁺BF₄⁻)

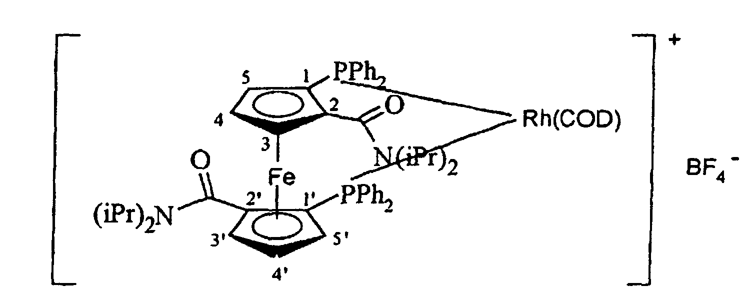

Zur klaren braunen Lösung von 100 mg (0.246 mmol) Bis(1,5-cyclooctadien)-rhodium(I)-tetrafluoroborat in 10 ml entgastem Dichlormethan wurden bei Raumtemperatur in einer Portion 197 mg (0.246 mmol) kristallines, homochirales C₂-symmetrisches 1,1'-Bis(diphenylphosphino)-2,2'-bis(N,N-diisopropylamido)ferrocen (aus Beispiel 5) gegeben, wobei ein Farbumschlag nach orange eintrat. Ein DC (Kieselgel; Laufmittel Dichlormethan/Methanol (5:1)) des Reaktionsgemisches nach 10 Min. zeigte vollständigen Umsatz der Verbindung der Formel la (R_{f} = 0.94) und des [Rh(COD)₂]BF₄ (R_{f} = 0.00) zum Produkt (R_{f} = 0.70) an. Nach 30 Min. wurde das Gemisch im Vakuum zur Trockne eingeengt. Der Rückstand wurde in 3 ml Methanol gelöst und das Produkt durch Zugabe von 30 ml Ether ausgefällt, abgesaugt, mit 10 ml Ether gewaschen und im Vakuum getrocknet. Ausbeute 186 mg (68 % d. Th.) gelbes Pulver, Schmp. (unter Argon in abgeschmolzener Kapillare) 225 °C (Zers.; Schwarzfärbung bei 150-190 °C), [α]_{D}²⁰ +156° (c = 0.125 in CH₂Cl₂).
¹H-NMR (500 MHz, CDCl₃): δ = 7.80-6.70 (m, 20H, 4 x C₆H₅), 5.49 (br s, 2H, =CH von COD), 4.86 (br s, 2H, =CH von COD), 3.85-4.70 (m, 2H, 3-, 3'-H), 3.40 (br s, 2H, 2 x CHMe₂), 3.30 (br s, 1H, 4-H), 3.00 (br s, 1H, 4'-H), 2.70 (br s, 1H, 5-H), 2.53 (br s, 1H, 5'-H), 2.20-2.42 (m, 6H, 2 x CH₂ von COD und 2 x CHMe₂), 1.98 (br s, 2H, CH₂ von COD), 1.75 (br s, 2H, CH₂ von COD), 1.25 (m, 6H, CH₃), 0.95-1.22 (m, 12H, CH₃), 0.30-0.80 (m, 6H, CH₃).
¹³C-NMR (125.77 MHz, CDCl₃, Protonen-breitbandentkoppelt): δ = 170.74 (2C, C=O), 134.16 und 134.05 (2 x s oder 1 x d, 4C, arom. C), 131.36 (s, 4C, arom. C), 131.12 (s, 4C, arom. C), 131.02 und 130.94 (2 x s oder 1 x d, 4C, arom. C), 128.84 und 128.76 (2 x s oder 1 x d, 4C, arom. C), 128.52 und 128.44 (2 x s oder 1 x d, 4C, arom. C), 108.70 (br s, 2C, =C von COD), 107.83 (br s, 2C, =C von COD), 81.86 (s, 2C, C-2,2'), 78.07 (s, 2C, C-1,1'), 74.69 (s, 2C, C-3,3'), 69.48 (s, 2C, C-4,4'), 66.23 (s, 2C, C-5,5'), 52.73 (s, 1C, NCH), 47.38 (s, 1C, NCH), 34.22 (s, 1C, CH₂ von COD), 29.87 (s, 1C, CH₂ von COD), 28.06 (s, 1C, CH₂ von COD), 25.84 (s, 1C, CH₂ von COD), 19.35 (s, 2C, CH₃), 19.23 (s, 4C, CH₃), 18.96 (s, 2C, CH₃).
³¹P-NMR (161.98 MHz, CDCl₃): δ = +24.20 (d, ¹J_{P,Rh} = 149.6 Hz; zwei isochrone P-Atome, d. h. C₂-Symmetrie erhalten).
IR (KBr): v = 2971, 1628 (C=C oder C=O), 1548 (N-C=O), 1437, 1370, 1055, 697, 520 cm⁻¹.
MS (FAB, NBA): m/z (%) = 1022 (6), 1021 (22), 1020 (66), 1019 (100), 1018 (4), 1017 (7) (Peakserie für M⁺ des Kations (M-BF₄⁻); Intensitätsverhältnis identisch mit Isotopensimulation für C₅₆H₆₆FeP₂N₂O₂Rh); 913 (16), 912 (51), 911 (83), 910 (7), 909 (7) (Peakserie für M-BF₄⁻-COD).

### Beispiel 12

### Ruthenium(II)-iodid-cumol - Komplex des C₂-symmetrischen 1,1'-Bis(diphenylphosphino)-2,2'-bis(N,N-diisopropylamido)ferrocens (Formel Ia · Rul₂ · p-Me₂CH-C₆H₄-Me, R¹: Ph, (R²)₂: =O, R³: iPr); Herstellung aus dem entsprechenden homochiralen Diphosphin der Formel la und [p-Cumol-Rutheniumdiiodid]-Dimer

[p-Cumol-Rutheniumdiiodid]-Dimer wurde gemäß K. Mashima et al., J. Chem. Soc., Chem. Commun. 1989, 1208 aus Ruthenium(III)-chlorid Trihydrat, (R)-(-)-α-Phellandren und Kaliumiodid in wäßrigem Ethanol als purpurbrauner Feststoff hergestellt. 91 mg (0.0927 mmol) dieses Komplexes und 150 mg (0.185 mmol) kristallines, homochirales 1,1'-Bis(diphenylphosphino)-2,2'-bis(N,N-diisopropylamido)ferrocen (aus Beispiel 5) wurden in 8 ml Dichlormethan gelöst und die dunkle Lösung wurde bei Raumtemperatur gerührt. Ein DC (Dichlormethan/Methanol (15:1)) nach 10 Min. zeigte vollständigen Umsatz des Ruthenium-Komplexes und weitgehenden Umsatz des Diphosphins der Formel la an. Nach 1 Std. wurde das Lösungsmittel im Vakuum entfernt, der Rückstand in 3 ml Dichlormethan aufgenommen und das Produkt durch Zugabe von 20 ml Diethylether ausgefällt. Es wurde abgesaugt, mit Ether gewaschen und im Hochvakuum getrocknet. Ausbeute 174 mg hellbraunes Pulver (0.134 mmol, 73 % d.Th.), Schmp. ∼190 °C (Zers.; Schwarzfärbung ab 145 °C), [α]_{D}²⁰ = +280° (c = 0.1 in CH₂Cl₂). Das gefällte Produkt ist homogen gemäß DC (R_{f} = 0.30) und es ist kein freies Diphosphin der Formel la (R_{f} = 0.94) mehr detektierbar. ¹H- und ³¹P-NMR-Spektren dieses Produkts in CDCl₃ sind sehr komplex und verändern sich beim Stehen der NMR-Lösung nicht. NMR-Spektren in Toluol-d₈ oder Benzol-d₆ sind sofort nach dem Lösen der Probe ebenfalls sehr komplex, vereinfachen sich beim Stehen der NMR-Lösung bei Raumtemperatur aber zunehmend und ergeben nach 4 Tagen ein interpretierbares Spektrum:
³¹P-NMR (161.98 MHz, Toluol-d₈): nur noch ein Singulett bei δ = + 22.43; zwei isochrone P-Atome, d.h. C₂-Symmetrie erhalten und keine tetraedrische Ligandenanordnung am Ruthenium.
Das ¹H-NMR zeigt zwei Signalsätze für die Isopropylgruppe und die Methylgruppe des Cumol-Liganden, was auf das Vorliegen von zwei Konformeren (Verhältnis 1.6 : 1) des Cumol-Liganden hinweist. ¹H-NMR (400 MHz, Toluol-d₈): δ = 7.75 (m, 4H, C₆H₅), 7.10 (t, 8H, C₆H₅), 7.02 (t, 4H, C₆H₅), 6.98 (t, 3H, C₆H₅), 5.61 (br s, 2H, arom. H von Cumol), 5.36 (br s, 2H, arom. H von Cumol), 5.00 (d, ³J_{H,P} = 5.8 Hz, 1H, 5-H), 4.83 (d, ³J_{H,P} = 5.8 Hz, 1H, 5'-H), 3.92 (br s, 2H, 3,3'-H), 3.84 (br s, 2H, NCH), 2.95 (br s, 2H, NCH), 2.86 und 2.72 (2 x sept, ³J_{H,H} = 7 Hz, zusammen 1 H, Me₂CH-Aryl, 2 Konformere), 2.16 (s, 2H, 4,4'-H), 2.08 und 1.95 (2 x s, zusammen 3H, CH₃-Aryl, 2 Konformere), 1.47 (br d, 6H, CH₃), 1.27 (br d, 6H, CH₃), 1.17 und 1.00 (2 x d, ³J_{H,H} = 7 Hz, zusammen 6H, (CH₃)₂CH-Aryl, 2 Konformere), 0.85 (br d, 6H, CH₃), 0.48 (br d, 6H, CH₃).
IR (KBr): v = 1625, 1539, 1438, 1370, 1328, 699 cm⁻¹.
MS (FBA, CH₂Cl₂, NBA): m/z (%) = 1175 (10), 1174 (30), 1173 (58), 1172 (56), 1171 (100), 1170 (62), 1169 (53), 1168 (32), 1167 (9), 1166 (9) (Peakserie für M⁺ des Kations [iPrC₆H₄Me · RuI · Ia]⁺ ; Intensitätsverhältnis identisch mit Isotopensimulation für C₅₈H₆₈FeP₂N₂O₂IRu).

### Beispiel 13

### 1,5-Cyclooctadien-iridium(I)-chlorid-Komplex des C₂-symmetrischen 1,1'-Bis(diphenylphosphino)-2,2'-bis(N,N-diisopropylamido)ferrocens (Formel [Ia · Ir(I)(COD)Cl], R¹: Ph, (R²)₂: =O, R³: iPr); Herstellung aus dem entsprechenden homochiralen Diphosphin der Formel Ia und [Chloro-(1,5-cyclooctadien)-iridium(I)]-Dimer ([Ir(I)(COD)Cl]₂)

297 mg (0.44 mmol) [Chloro-(1,5-cyclooctadien)-iridium(I)]-Dimer (Strem Chemicals) und 716 mg (0.89 mmol) kristallines, homochirales 1,1'-Bis(diphenylphosphino)-2,2'bis(N,N-diisopropylamido)ferrocen (aus Beispiel 5) wurden in 40 ml Dichlormethan gelöst, das zuvor mittels durchperlendem Argon vom gelösten Sauerstoff befreit worden war. Die klare Lösung wurde 15 Min. unter Argon bei 20 °C gerührt. Das Lösungsmittel wurde im Vakuum entfernt und der Rückstand im Hochvakuum getrocknet. Man erhielt 1.01 g (100 % d. Th.) orangefarbenes Pulver.
¹H-NMR (250 MHz, CDCl₃): δ = 7.91 (br s, 4H, arom. H), 7.70 (br s, 4H, arom. H), 7.28-7.52 (m, 12H, arom. H), 4.40-4.87 (m, 6H, 2 =CH von COD, 4 Ferrocenyl-H), 4.14 (br s, 4H, 2 =CH von COD, 2 Ferrocenyl-H), 3.30 (br s, 2H, NCH), 3.15 (br s, 2H, NCH), 2.47 (m, 2H, CH₂ von COD), 2.01 (m, 2H, CH₂ von COD), 1.58-1.79 (m, 4H, CH₂ von COD), 1.36 (d, 6H, CH₃), 1.25 (br s, 6H, CH₃), 1.15 (d, 6H, CH₃), 0.96 (br s, 6H, CH₃).
³¹P-NMR (161.98 MHz, CDCl₃): δ = + 21.60 (Singulett, Halbwertsbreite 106 Hz). MS (FAB, NBA): m/z (%) = 1112 (5), 1111 (19), 1110 (60), 1109 (100), 1108 (38), 1107 (58), 1106 (2), 1105 (3) (Peakserie für M-CI; Intensitätsverhältnis in Übereinstimmung mit Isotopensimulation für C₅₆H₆₆FeP₂N₂O₂Ir); 1003 (5), 1002 (12), 1001 (14), 1000 (12), 999 (13), 998 (4), 997 (4) (Peakserie für M-Cl-COD).

## Patentansprüche

1. Verbindungen der Formel I, 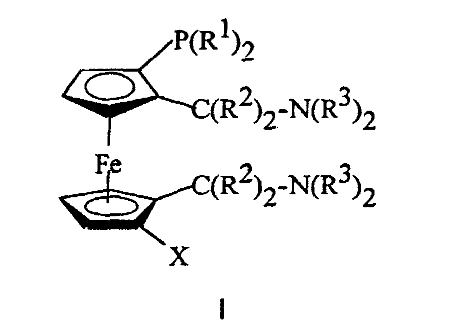 worin
die Substituenten R¹ Cyclohexyl, unsubstituiertes Phenyl C₆H₅ oder substituiertes Phenyl C₆H₅₋ₙR⁴ₙ bedeuten, wobei n für 1 bis 5 steht und die Substituenten R⁴ unverzweigtes oder verzweigtes C₁- bis C₄-Alkyl, C₁- bis C₄-Alkoxy oder Halogen bedeuten;
die beiden geminalen Substituenten R² zusammen ein doppelt gebundenes Sauerstoffatom bedeuten (d. h. (R²)₂ bedeutet =O) oder jeder Substituent R² für sich Wasserstoff bedeutet;
die Substituenten R³ jeder für sich unverzweigtes oder verzweigtes C₁- bis C₄-Alkyl, Cyclopentyl, Cyclohexyl, unsubstituiertes Phenyl C₆H₅ oder substituiertes Phenyl C₆H₅₋ₙR⁴ₙ bedeuten, wobei n und R⁴ die oben unter R¹ angegebenen Bedeutungen haben, oder die Substituenten R³ miteinander unter Ausbildung eines Ringes verbunden sind, wobei sie zusammen Tetramethylen -(CH₂)₄-, Pentamethylen -(CH₂)₅-, 3-Oxa-pentamethylen -(CH₂)₂-O-(CH₂)₂- oder N-Methyl-3-azapentamethylen -(CH₂)₂-N(CH₃)-(CH₂)₂- bedeuten;
der Substituent X Wasserstoff oder P(R¹)₂ bedeutet; und ihre Salze.

2. Verbindungen der Formel I gemäß Anspruch 1, in der
die Substituenten R¹ Cyclohexyl, Phenyl, p-Tolyl, p-tert-Butylphenyl oder p-Halogenophenyl bedeuten, wobei Halogen hier für Fluor, Chlor oder Brom steht;
die beiden Substituenten R² zusammen ein doppelt gebundenes Sauerstoffatom bedeuten (d. h. (R²)₂ bedeutet =O) oder jeder Substituent R² für sich Wasserstoff bedeutet;
die Substituenten R³ Isopropyl, Cyclohexyl oder Phenyl bedeuten oder die beiden Substituenten R³ zusammen für -(CH₂)₄-, -(CH₂)₅-, -(CH₂)₂-O-(CH₂)₂- oder -(CH₂)₂-N(CH₃)-(CH₂)₂- stehen;
der Substituent X Wasserstoff oder P(R¹)₂ bedeutet, wobei R¹ für Cyclohexyl, Phenyl, p-Tolyl, p-tert-Butylphenyl oder p-Halogenophenyl steht und Halogen hier für Fluor, Chlor oder Brom steht.

3. Verbindungen der Formel I gemäß den Ansprüchen 1 und/oder 2, in der R¹ für Phenyl steht, die beiden Substituenten R² zusammen für ein doppelt gebundenes Sauerstoffatom stehen, R³ für Isopropyl steht und X für Wasserstoff oder P(Phenyl)₂ steht.

4. Verbindungen der Formel I gemäß einem oder mehreren der Ansprüche 1 bis 3 in optisch aktiver Form.

5. Verbindungen der Formel I gemäß einem oder mehreren der Ansprüche 1 bis 3 in racemischer Form.

6. Verfahren zur Herstellung von optisch aktiven Monophosphinen der Formel I gemäß einem oder mehreren der Ansprüche 1 bis 4, in der die Reste R¹, R² und R³ die in den Ansprüchen 1 bis 4 angegebenen Bedeutungen haben und X für Wasserstoff steht, **dadurch gekennzeichnet, daß** man Verbindungen der Formel III, 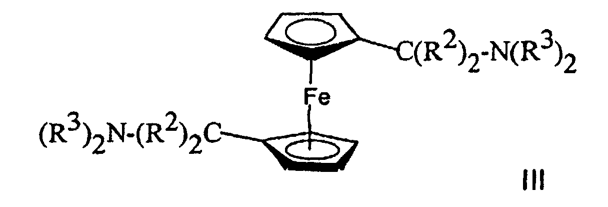 in der die Reste R² und R³ die in den Ansprüchen 1 bis 4 angegebenen Bedeutungen haben, mit n-Butyllithium in Gegenwart eines homochiralen tertiären Amins asymmetrisch mono-ortho-lithiiert und die chirale Monolithiumverbindung in situ mit einem Chlorphosphin der Formel CIP(R¹)₂ umsetzt, in der die Reste R¹ die in den Ansprüchen 1 bis 4 angegebene Bedeutung haben.

7. Verfahren zur Herstellung von optisch aktiven C₂-symmetrischen Diphosphinen der Formel I gemäß einem oder mehreren der Ansprüche 1 bis 4, in der die Reste R¹, R² und R³ die in den Ansprüchen 1 bis 4 angegebenen Bedeutungen haben und X für P(R¹)₂ steht, **dadurch gekennzeichnet, daß** man die optisch aktiven Monophosphine der Formel I, in der die Reste R¹, R² und R³ die in den Ansprüchen 1 bis 4 angegebenen Bedeutungen haben und X für Wasserstoff steht, mit n-Butyllithium in Gegenwart eines homochiralen tertiären Amins asymmetrisch mono-ortho-lithiiert und die chirale Monolithiumverbindung in situ mit einem Chlorphosphin der Formel CIP(R¹)₂ umsetzt, in der die Reste R¹ die in den Ansprüchen 1 bis 4 angegebene Bedeutung haben.

8. Verfahren zur Herstellung von optisch aktiven C₂-symmetrischen Diphosphinen der Formel I gemäß einem oder mehreren der Ansprüche 1 bis 4, in der die Reste R¹, R² und R³ die in den Ansprüchen 1 bis 4 angegebenen Bedeutungen haben und X für P(R¹)₂ steht, **dadurch gekennzeichnet, daß** man eine Verbindung der Formel III, 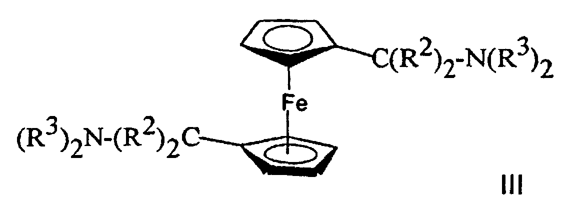 in der R² und R³ die in den Ansprüchen 1 bis 4 angegebenen Bedeutungen haben, zunächst mit n-Butyllithium in Gegenwart eines homochiralen tertiären Amins deprotoniert, dann durch Zusatz eines Chlorphosphins der Formel CIP(R¹)₂, in der R¹ die in den Ansprüchen 1 bis 4 angegebene Bedeutung hat, phosphinyliert, dann ohne Isolierung des entstandenen Monophosphins durch erneuten Zusatz von n-Butyllithium den zweiten Deprotonierungsschritt ausführt und schließlich durch erneuten Zusatz des Chlorphosphins der Formel CIP(R¹)₂ die zweite Phosphinylierung bewirkt.

9. Verfahren zur Herstellung von racemischen Verbindungen der Formel I gemäß einem oder mehreren der Ansprüche 1 bis 3 und 5, in der R¹, R², R³ und X die in den Ansprüchen 1 bis 3 und 5 angegebenen Bedeutungen haben, **dadurch gekennzeichnet, daß** man achirale Ferrocene der Formel III, 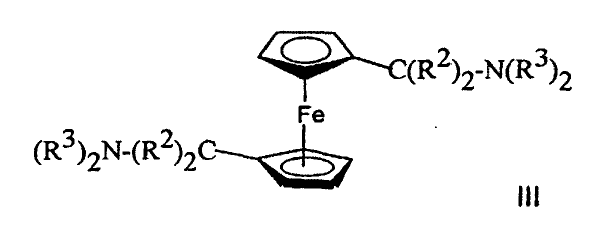 in der die Reste R² und R³ die in den Ansprüchen 1 bis 3 und 5 angegebenen Bedeutungen haben, oder racemische Monophosphine der Formel I, in der R¹, R² und R³ die in den Ansprüchen 1 bis 3 und 5 angegebenen Bedeutungen haben und X für Wasserstoff steht, mit n-Butyllithium in Gegenwart eines racemischen oder achiralen tertiären Amins symmetrisch mono-ortho-lithiiert und die Monolithiumverbindung in situ mit einem Chlorphosphin der Formel CIP(R¹)₂ umsetzt, in der die Reste R¹ die in den Ansprüchen 1 bis 3 und 5 angegebene Bedeutung haben, oder, im Falle der Herstellung von Diphosphinen aus den Verbindungen der Formel III, schrittweise symmetrisch dilithiiert/diphosphinyliert.

10. Verfahren gemäß Anspruch 9, **dadurch gekennzeichnet, daß** als Amin N,N,N',N'-Tetramethylethylendiamin eingesetzt wird.

11. Verwendung von Verbindungen der Formel I gemäß einem oder mehreren der Ansprüche 1 bis 5 als Liganden in Übergangsmetallkomplexen.

12. Übergangsmetallkomplexe, enthaltend mindestens einen Liganden der Formel I gemäß einem oder mehreren der Ansprüche 1 bis 5.

13. Übergangsmetallkomplexe gemäß Anspruch 12 der Übergangsmetalle Ruthenium, Rhodium, Iridium, Nickel, Palladium, Platin und Silber.

14. Verwendung von Übergangsmetallkomplexen gemäß Anspruch 12 und/oder 13 als Katalysatoren.

15. Verwendung von Übergangsmetallkomplexen gemäß Anspruch 13 zur asymmetrischen Katalyse.

16. Verbindungen der Formel II, 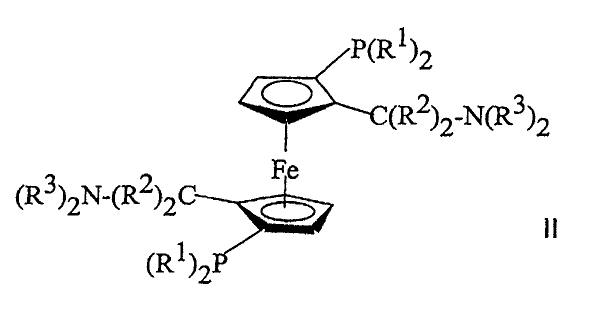 worin
die Substituenten R¹ Cyclohexyl, unsubstituiertes Phenyl C₆H₅ oder substituiertes Phenyl C₆H₅₋ₙR⁴ₙ bedeuten, wobei n für 1 bis 5 steht und die Substituenten R⁴ unverzweigtes oder verzweigtes C₁- bis C₄-Alkyl, C₁- bis C₄-Alkoxy oder Halogen bedeuten;
die beiden geminalen Substituenten R² zusammen ein doppelt gebundenes Sauerstoffatom bedeuten (d. h. (R²)₂ bedeutet =O) oder jeder Substituent R² für sich Wasserstoff bedeutet;
die Substituenten R³ jeder für sich unverzweigtes oder verzweigtes C₁- bis C₄-Alkyl, Cyclopentyl, Cyclohexyl, unsubstituiertes Phenyl C₆H₅ oder substituiertes Phenyl C₆H₅₋ₙR⁴ₙ bedeuten, wobei n und R⁴ die oben unter R¹ angegebenen Bedeutungen haben, oder die Substituenten R³ miteinander unter Ausbildung eines Ringes verbunden sind, wobei sie zusammen Tetramethylen -(CH₂)₄-, Pentamethylen -(CH₂)₅-, 3-Oxa-pentamethylen -(CH₂)₂-O-(CH₂)₂- oder N-Methyl-3-azapentamethylen -(CH₂)₂-N(CH₃)-(CH₂)₂- bedeuten; und ihre Salze.

17. Verfahren zur Herstellung von Verbindungen der Formel II, in der die Reste R¹, R² und R³ die in Anspruch 16 angegebenen Bedeutungen haben, **dadurch gekennzeichnet, daß** man Verbindungen der Formel III, 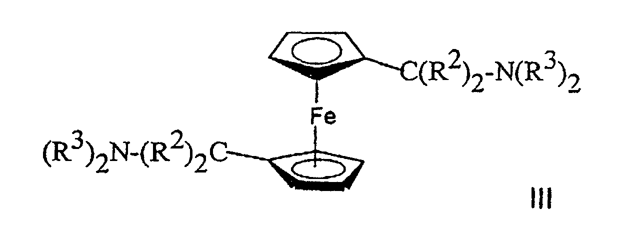 in der die Reste R² und R³ die in Anspruch 16 angegebenen Bedeutungen haben, mit sec-Butyllithium in Gegenwart eines tertiären Amins ortho-lithiiert und die Dilithiumverbindung in situ mit einem Chlorphosphin der Formel CIP(R¹)₂ umsetzt, in der die Reste R¹ die in Anspruch 16 angegebene Bedeutung haben.

18. Verfahren gemäß Anspruch 17, **dadurch gekennzeichnet, daß** als Amin N,N,N',N'-Tetramethylethylendiamin eingesetzt wird.

19. Verwendung von Verbindungen der Formel II gemäß Anspruch 16 als Liganden in Übergangsmetallkomplexen.

20. Übergangsmetallkomplexe, enthaltend mindestens einen Liganden der Formel II gemäß Anspruch 16.

21. Verwendung von Übergangsmetallkomplexen gemäß Anspruch 20 als Katalysatoren.

## Claims

1. A compound of the formula I 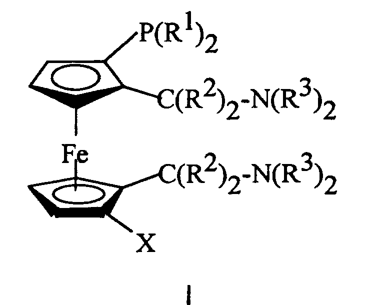 in which
the substituents R¹ are cyclohexyl, unsubstituted phenyl C₆H₅ or substituted phenyl C₆H₅₋ₙR⁴ₙ, where n is 1 to 5 and the substituents R⁴ are unbranched or branched C₁- to C₄-alkyl, C₁- to C₄-alkoxy or halogen;
the two geminal substituents R² together are a doubly bonded oxygen atom (i.e. (R²)₂ is =O) or each substituent R² on its own is hydrogen;
the substituents R³, each on their own, are unbranched or branched C₁- to C₄-alkyl, cyclopentyl, cyclohexyl, unsubstituted phenyl C₆H₅ or substituted phenyl C₆H₅₋ₙR⁴ₙ, where n and R⁴ are as defined above under R¹, or the substituents R³ are connected to one another to form a ring, in which case together they are tetramethylene-(CH₂)₄-, pentamethylene-(CH₂)₅-, 3-oxapentamethylene-(CH₂)₂-O-(CH₂)₂- or N-methyl-3-azapentamethylene-(CH₂)₂-N(CH₃)-(CH₂)₂-;
the substituent X is hydrogen or P(R¹)₂; or a salt thereof.

2. A compound of the formula I as claimed in claim 1, in which
the substituents R¹ are cyclohexyl, phenyl, p-tolyl, p-tert-butylphenyl or p-halophenyl, in which case halogen here is fluorine, chlorine or bromine;
the two substituents R² together are a doubly bonded oxygen atom (i.e. (R²)₂ is =O) or each substituent R² by itself is hydrogen;
the substituents R³ are isopropyl, cyclohexyl or phenyl, or the two substituents R³ together are -(CH₂)₄-, -(CH₂)₅-, -(CH₂)₂-O-(CH₂)₂- or -(CH₂)₂-N(CH₃)-(CH₂)₂-;
the substituent X is hydrogen or P(R¹)₂ in which R¹ is cyclohexyl, phenyl, p-tolyl, p-tert-butylphenyl or p-halophenyl and halogen in this case is fluorine, chlorine or bromine.

3. A compound of the formula I as claimed in claim 1 and/or 2, in which R¹ is phenyl, the two substituents R² together are a doubly bonded oxygen atom, R³ is isopropyl and X is hydrogen or P(phenyl)₂.

4. A compound of the formula I as claimed in one or more of claims 1 to 3 in optically active form.

5. A compound of the formula I as claimed in one or more of claims 1 to 3 in racemic form.

6. A process for preparing an optically active monophosphine of the formula I as claimed in one or more of claims 1 to 4, in which the radicals R¹, R² and R³ are as defined in claims 1 to 4 and X is hydrogen, which comprises subjecting compounds of the formula III, 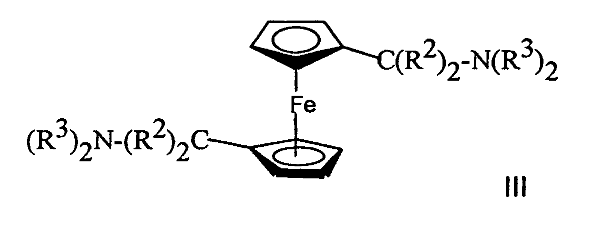 in which the radicals R² and R³ are as defined in claims 1 to 4, to asymmetric mono-ortho-lithiation using n-butyllithium in the presence of a homochiral tertiary amine, and reacting the chiral monolithium compound in situ with a chlorophosphine of the formula CIP(R¹)₂ in which the radicals R¹ are as defined in claims 1 to 4.

7. A process for preparing an optically active C₂-symmetric diphosphine of the formula I as claimed in one or more of claims 1 to 4, in which the radicals R¹, R² and R³ are as defined in claims 1 to 4 and X is P(R¹)₂, which comprises subjecting an optically active monophosphine of the formula I in which the radicals R¹, R² and R³ are as defined in claims 1 to 4 and X is hydrogen to asymmetric mono-ortho-lithiation with n-butyllithium in the presence of a homochiral tertiary amine, and reacting the chiral monolithium compound in situ with a chlorophosphine of the formula CIP(R¹)₂ in which the radicals R¹ are as defined in claims 1 to 4.

8. A process for preparing an optically active C₂-symmetric diphosphine of the formula I as claimed in one or more of claims 1 to 4, in which the radicals R¹, R² and R³ are as defined in claims 1 to 4 and X is P(R¹)₂, which comprises subjecting a compound of the formula III 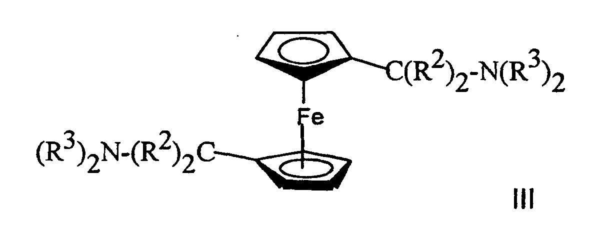 in which R² and R³ are as defined in claims 1 to 4 first of all to deprotonation with n-butyllithium in the presence of a homochiral tertiary amine, then to phosphinylation by adding a chlorophosphine of formula ClP(R¹)₂ in which R¹ is as defined in claims 1 to 4, then carrying out the second deprotonation step by renewed addition of n-butyllithium, without isolating the monophosphine formed, and, finally, bringing about the second phosphinylation by renewed addition of chlorophosphine of the formula ClP(R¹)₂.

9. A process for preparing a racemic compound of the formula I as claimed in one or more of claims 1 to 3 and 5, in which R¹, R², R³ and X are as defined in claims 1 to 3 and 5, which comprises subjecting an achiral ferrocene of the formula III 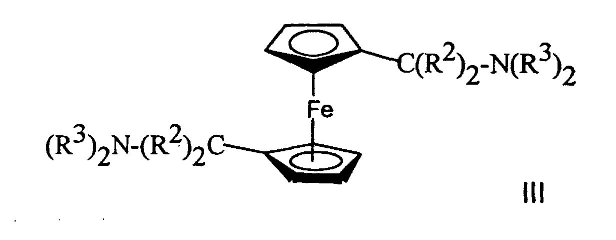 in which the radicals R² and R³ are as defined in claims 1 to 3 and 5, or a racemic monophosphine of the formula I in which R¹, R² and R³ are as defined in claims 1 to 3 and 5 and X is hydrogen, to symmetric mono-ortho-lithiation with n-butyllithium in the presence of a racemic or achiral tertiary amine, and reacting the monolithium compound in situ with a chlorophosphine of the formula ClP(R¹)₂ in which the radicals R¹ are as defined in claims 1 to 3 and 5, or, in the case of the preparation of a diphosphine from a compound of the formula III, carrying out stepwise symmetric dilithiation/diphosphinylation.

10. The process as claimed in claim 9, wherein the amine employed is N,N,N',N'-tetramethylethylenediamine.

11. The use of a compound of the formula I as claimed in one or more of claims 1 to 5 as a ligand in transition metal complexes.

12. A transition metal complex comprising at least one ligand of the formula I as claimed in one or more of claims 1 to 5.

13. A transition metal complex as claimed in claim 12 of the transition metal ruthenium, rhodium, iridium, nickel, palladium, platinum or silver.

14. The use of a transition metal complex as claimed in claim 12 and/or 13 as a catalyst.

15. The use of a transition metal complex as claimed in claim 13 for asymmetric catalysis.

16. A compound of the formula II 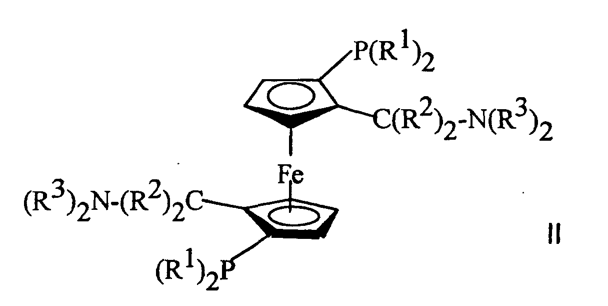 in which
the substituents R¹ are cyclohexyl, unsubstituted phenyl C₆H₅ or substituted phenyl C₆H₅₋ₙR⁴ₙ, where n is 1 to 5 and the substituents R⁴ are unbranched or branched C₁- to C₄-alkyl, C₁- to C₄-alkoxy or halogen;
the two geminal substituents R² together are a doubly bonded oxygen atom (i.e. (R²)₂ is =O) or each substituent R² on its own is hydrogen;
the substituents R³, each on their own, are unbranched or branched C₁- to C₄-alkyl, cyclopentyl, cyclohexyl, unsubstituted phenyl C₆H₅ or substituted phenyl C₆H₅₋ₙR⁴ₙ, where n and R⁴ are as defined above under R¹, or the substituents R³ are connected to one another to form a ring, in which case together they are tetramethylene-(CH₂)₄-, pentamethylene- (CH₂)₅-, 3-oxapentamethylene-(CH₂)₂-O-(CH₂)₂- or N-methyl-3-azapentamethylene-(CH₂)₂-N(CH₃)-(CH₂)₂-; or a salt thereof.

17. A process for preparing a compound of the formula II in which the radicals R¹, R² and R³ are as defined in claim 16, which comprises subjecting a compound of the formula III 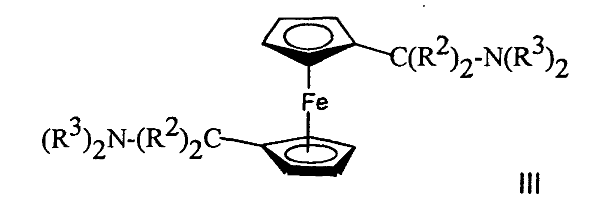 in which the radicals R² and R³ are as defined in claim 16 to ortho-lithiation with sec-butyllithium in the presence of a tertiary amine, and reacting the dilithium compound in situ with a chlorophosphine of the formula ClP(R¹)₂ in which the radicals R¹ are as defined in claim 16.

18. The process as claimed in claim 17, wherein the amine employed is N,N,N',N'-tetramethylethylenediamine.

19. The use of a compound of the formula II as claimed in claim 16 as a ligand in transition metal complexes.

20. A transition metal complex comprising at least one ligand of the formula II as claimed in claim 16.

21. The use of a transition metal complex as claimed in claim 20 as a catalyst.

## Revendications

1. Composés de formule I 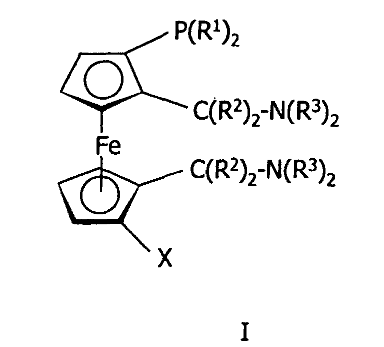 où
les substituants R¹ représentent cyclohexyle, phényle non substitué C₆H₅ ou phényle substitué C₆H₅₋ₙR⁴ₙ, où n représente 1 à 5 et les substituants R⁴ représentent alkyle en C₁ à C₄ non ramifié ou ramifié, alcoxy en C₁ à C₄ ou halogène ;
les deux substituants géminés R² représentent ensemble un atome d'oxygène doublement lié (c'est-à-dire que (R²)₂ représente =O) ou chaque substituant R² représente l'hydrogène ;
les substituants R³ représentent chacun alkyle en C₁ à C₄ non ramifié ou ramifié, cyclopentyle, cyclohexyle, phényle non substitué C₆H₅ ou phényle substitué C₆H₅₋ₙR⁴ₙ, où n et R⁴ ont les significations indiquées ci-dessus au sujet de R¹, ou bien les substituants R³ sont liés entre eux en formant un cycle, où ils représentent ensemble tétraméthylène -(CH₂)₄-, pentaméthylène -(CH₂)₅-, 3-oxa-pentaméthylène -(CH₂)₂-O-(CH₂)₂- ou N-méthyl-3-azapentaméthylène -(CH₂)₂-N(CH₃)-(CH₂)₂- ;
le substituant X représente l'hydrogène ou P(R¹)₂;
et leurs sels.

2. Composés de formule I selon la revendication 1, où les substituants R¹ représentent cyclohexyle, phényle, p-tolyle, p-tert-butylphényle ou p-halogénophényle, où halogène représente ici le fluor, le chlore ou le brome ;
les deux substituants R² représentent ensemble un atome d'oxygène doublement lié (c'est-à-dire que (R²)₂ représente =O) ou bien chaque substituant R² représente l'hydrogène ;
les substituants R³ représentent isopropyle, cyclohexyle ou phényle ou bien les deux substituants R³ représentent ensemble -(CH₂)₄-, -(CH₂)₅-, -(CH₂)₂-O-(CH₂)₂- ou -(CH₂)₂-N(CH₃)-(CH₂)₂-;
le substituant X représente l'hydrogène ou P(R¹)₂, où R¹ représente cyclohexyle, phényle, p-tolyle, p-tert-butylphényle ou p-halogénophényle et halogène représente ici le fluor, le chlore ou le brome.

3. Composés de formule I selon les revendications 1 et/ou 2, où R¹ représente phényle, les deux substituants R² représentent ensemble un atome d'oxygène doublement lié, R³ représente isopropyle et X représente l'hydrogène ou P(phényle)₂.

4. Composés de formule I selon une ou plusieurs des revendications 1 à 3 sous forme optiquement active.

5. Composés de formule I selon une ou plusieurs des revendications 1 à 3 sous forme racémique.

6. Procédé de préparation de monophosphines optiquement actives de formule I selon une ou plusieurs des revendications 1 à 4 où les restes R¹, R² et R³ ont les significations indiquées dans les revendications 1 à 4 et X représente l'hydrogène, **caractérisé en ce que** l'on mono-ortho-lithie de manière asymétrique des composés de formule III 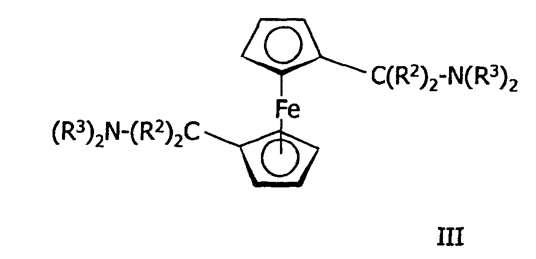 où les restes R² et R³ ont les significations indiquées dans les revendications 1 à 4 avec le n-butyllithium en présence d'une amine tertiaire homochirale et on fait réagir le composé de monolithium chiral in situ avec une chlorophosphine de formule CIP(R¹)₂ où les restes R¹ ont la signification indiquée dans les revendications 1 à 4.

7. Procédé de préparation de diphosphines C₂-symétriques optiquement actives de formule I selon une ou plusieurs des revendications 1 à 4 où les restes R¹, R² et R³ ont les significations indiquées dans les revendications 1 à 4 et X représente P(R¹)₂, **caractérisé en ce que** l'on mono-ortho-lithie de manière asymétrique les monophosphines optiquement actives de formule I où les restes R¹, R² et R³ ont les significations indiquées dans les revendications 1 à 4 et X représente l'hydrogène avec le n-butyllithium en présence d'une amine tertiaire homochirale et on fait réagir le composé de monolithium chiral in situ avec une chlorophosphine de formule CIP(R¹)₂ où les restes R¹ ont la signification indiquée dans les revendications 1 à 4.

8. Procédé de préparation de diphosphines C₂-symétriques optiquement actives de formule I selon une ou plusieurs des revendications 1 à 4 où les restes R¹, R² et R³ ont les significations indiquées dans les revendications 1 à 4 et X représente P(R¹)₂, **caractérisé en ce que** l'on déprotone d'abord un composé de formule III 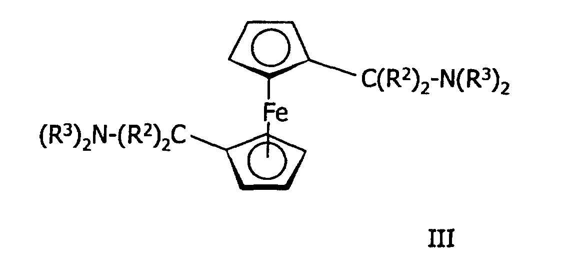 où R² et R³ ont les significations indiquées dans les revendications 1 à 4 avec le n-butyllithium en présence d'une amine tertiaire homochirale, après quoi on le phosphinyle par addition d'une chlorophosphine de formule CIP(R¹)₂ où R¹ a la signification indiquée dans les revendications 1 à 4, puis, sans isolement de la monophosphine formée, on réalise la deuxième étape de déprotonation par une nouvelle addition de n-butyllithium et enfin on réalise la seconde phosphinylation par une nouvelle addition de chlorophosphine de formule CIP(R¹)₂.

9. Procédé de préparation de composés racémiques de formule I selon une ou plusieurs des revendications 1 à 3 et 5, où R¹, R², R³ et X ont les significations indiquées dans les revendications 1 à 3 et 5, **caractérisé en ce que** l'on mono-ortho-lithie de manière symétrique des ferrocènes achiraux de formule III 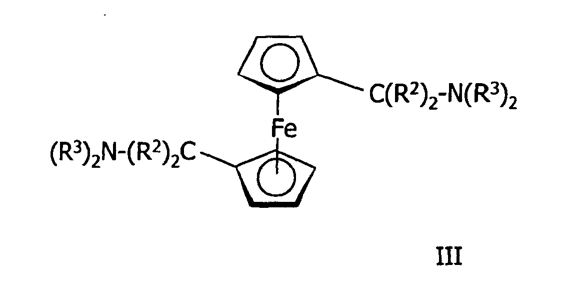 où les restes R² et R³ ont les significations indiquées dans les revendications 1 à 3 et 5 ou des monophosphines racémiques de formule I où R¹, R² et R³ ont les significations indiquées dans les revendications 1 à 3 et 5 et X représente l'hydrogène avec le n-butyllithium en présence d'une amine tertiaire racémique ou achirale et on fait réagir le composé de monolithium in situ avec une chlorophosphine de formule CIP(R¹)₂ où les restes R¹ ont la signification indiquée dans les revendications 1 à 3 et 5 ou bien, dans le cas de la préparation de diphosphines à partir des composés de formule III, on dilithle/dlphosphinyle de manière symétrique par étapes.

10. Procédé selon la revendication 9, **caractérisé en ce que** l'on utilise la N,N,N',N'-tétraméthyléthylènediamine comme amine.

11. Utilisation de composés de formule I selon une ou plusieurs des revendications 1 à 5 comme ligands dans des complexes de métaux de transition.

12. Complexes de métaux de transition contenant au moins un ligand de formule I selon ou une plusieurs des revendications 1 à 5.

13. Complexes de métaux de transition selon la revendication 12 des métaux de transition ruthénium, rhodium, iridium, nickel, palladium, platine et argent.

14. Utilisation de complexes de métaux de transition selon la revendication 12 et/ou 13 comme catalyseurs.

15. Utilisation de complexes de métaux de transition selon la revendication 13 pour la catalyse asymétrique.

16. Composés de formule II 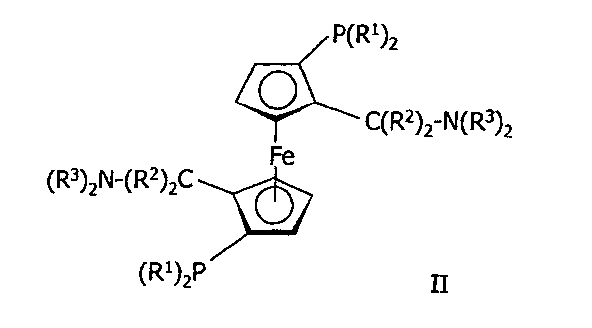 où
les substituants R¹ représentent cyclohexyle, phényle non substitué C₆H₅ ou phényle substitué C₆H₅₋ₙR⁴ₙ où n représente 1 à 5 et les substituants R⁴ représentent alkyle en C₁ à C₄ non ramifié ou ramifié, alcoxy en C₁ à C₄ ou halogène ;
les deux substituants géminés R² représentent ensemble un atome d'oxygène doublement lié (c'est-à-dire que (R²)₂ représente =O) ou chaque substituant R² représente l'hydrogène ;
les substituants R³ représentent chacun alkyle en C₁ à C₄ non ramifié ou ramifié, cyclopentyle, cyclohexyle, phényle non substitué C₆H₅ ou phényle substitué C₆H₅₋ₙR⁴ₙ où n et R⁴ ont les significations indiquées ci-dessus au sujet de R¹, ou bien les substituants R³ sont liés entre eux en formant un cycle, où ils signifient ensemble tétraméthylène -(CH₂)₄-, pentaméthylène -(CH₂)₅-, 3-oxa-pentaméthylène -(CH₂)₂-O-(CH₂)₂- ou N-méthyl-3-azapentaméthylène -(CH₂)₂-N(CH₃)-(CH₂)₂-;
et leurs sels.

17. Procédé de préparation de composés de formule II où les restes R¹, R² et R³ ont les significations indiquées dans la revendication 16, **caractérisé en ce que** l'on ortho-lithie des composés de formule III 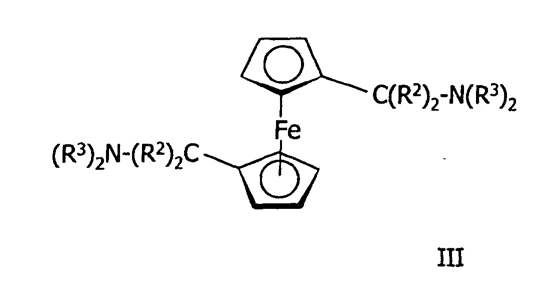 où les restes R² et R³ ont les significations indiquées dans la revendication 16 avec le sec-butyllithium en présence d'une amine tertiaire et on fait réagir le composé de dilithium in situ avec une chlorophosphine de formule CIP(R¹)₂ où les restes R¹ ont la signification indiquée dans la revendication 16.

18. Procédé selon la revendication 17, **caractérisé en ce que** l'on utilise la N,N,N',N'-tétraméthyléthylènediamine comme amine.

19. Utilisation de composés de formule II selon la revendication 16 comme ligands dans des complexes de métaux de transition.

20. Complexes de métaux de transition contenant au moins un ligand de formule II selon la revendication 16.

21. Utilisation de complexes de métaux de transition selon la revendication 20 comme catalyseurs.
